Europäisches Patentamt

European Patent Office

Office européen des brevets

(19)

(11) Publication number: **0 271 003**
**A2**

(12) # EUROPEAN PATENT APPLICATION

(21) Application number: 87117891.9

(22) Date of filing: 03.12.87

(51) Int. Cl.4: **C12N 15/00** , C12N 1/20 , C12N 5/00 , C12P 21/02

The applicant has filed a statement in accordance with Rule 28 (4) EPC (issue of a sample only to an expert). Accession number(s) of the deposit(s): DSM 4290, 4292, 4293, 4296.

Claims for the following Contracting States: ES + GR.

(30) Priority: 05.12.86 GB 8629153

(43) Date of publication of application:
15.06.88 Bulletin 88/24

(84) Designated Contracting States:
BE CH DE ES FR GB GR IT LI LU NL SE

(71) Applicant: **CIBA-GEIGY AG**
**Klybeckstrasse 141**
**CH-4002 Basel(CH)**

(72) Inventor: **Asselbergs, Fredericus A. M., Dr.**
**Rainallee 88/3**
**CH-4125 Riehen(CH)**
Inventor: **Alaimo, Danielle**
**Damberg Rue 30**
**F-68200 Brunstatt(FR)**

(74) Representative: **Zumstein, Fritz, Dr. et al**
**Bräuhausstrasse 4**
**D-8000 München 2(DE)**

(54) **Expression vectors.**

(57) Recombinant DNA molecules comprising a series of functional elements in the following order: (a) an enhancer/promoter unit linked operatively to (b) a DNA segment which can be transcribed into a mRNA having coding capacity for a desired protein product and comprising signals for polyadenylation and for efficient transportation to the cytoplasm, followed by a DNA segment (c) consisting a promoter unit ($c_1$) operatively linked to a DNA subsegment ($c_2$) which can be transcribed into the mRNA of a selection marker gene, wherein said promoter unit ($c_1$) lacks a functional enhancer but is capable of being stimulated by the enhancer located in (a) due to the absence of an enhancer stop DNA sequence between (a) and ($c_1$), are provided. The specific arrangement of the functional elements assures that a high proportion of transfected eukaryotic cells stably express the heterologous gene even when said DNA is applied to said eukaryotic cells as circular DNA.

EP 0 271 003 A2

Figure 1

- 1 -

4-16676/16199

## Expression vectors

This invention relates to uniquely reliable expression vectors that use a specific arrangement of an enhancer-containing genetic expression module for a desired gene product linked to an enhancer-less marker gene module, to assure that after introduction into a suitable host cell a high percentage of the marker expressing progeny cells also express the desired gene product.

## Background of the invention

Transformation is a commonly employed genetic engineering procedure by which new genetic material is transferred into eukaryotic or prokaryotic cells. Ordinarily, the number of cells in the population undergoing transformation and stably incorporating the exogenous DNA is quite low. The problem of this low transformation frequency by the exogenous DNA can be obviated by transforming the host cells with a selection marker (i.e. DNA expressing an easily identifiable gene, for example resistance to an antibiotic) along with the production gene (i.e. the gene the expression of which is the real aim of the experiment as it encodes a desired product, such as a useful enzyme, hormone or lymphokine). Some marker genes, designated recessive, can only be used in mutant host cell types. Upon transformation the cell population is examined for the presence of the marker. It depends on whether and how the selection marker is linked to the production gene, and what proportion of the cells expressing the marker also contain and express the production gene. Those cells in which the marker DNA has successfully been incorporated will exhibit the marker identity (e.g. survival in the presence of the antibiotic) and those cells that have failed to incorporate the marker will not exhibit the marker feature (e.g. they will die in medium with the antibiotic). Generally, only part of the cells exhibiting marker identity will also contain the production gene in a functional form. Vector constructs that maximize the probability that cells with marker identity also express the production gene constitute an improvement in the art.

A typical eukaryotic gene consists of three regions, i.e. the protein-encoding part proper plus a region containing mRNA processing signals and a region containing transcription control signals. These regions may overlap to some extent. The level of gene expression is primarily regulated by the level of transcription. The transcription control signals together fall into two categories: (1) upstream DNA sequences, located at or shortly upstream from the transcription start point, and (2) enhancers. Typical upstream elements found in many genes are a "TATA" box and a "CAAT" box located respectively approximately 30 and 80 nucleotides upstream from the transcription start site. [J. Corden et al., Science 209, 1406-1414 (1980); R. Breatnach and P. Chambon, Annual Review of Biochemistry 50, 349-383 (1981)]. A characteristic property of upstream sequences is that in order to be functional they have to be in a fixed orientation at a fixed distance from the transcription start site. Upstream elements together are frequently referred to as the promoter as opposed to enhancer elements. These enhancers can work over longer, variable distances in either orientation. Enhancers are frequently located upstream from the transcription start site but can also operate when located downstream and can even activate multiple genes [F. Asselbergs et al., J. Mol. Biol. 189, 401-411 (1986)]. The operating mechanism of the enhancers is not completely understood. An important phenomenon concerning enhancer function is that certain DNA sequences, frequently of prokaryotic origin, when interposed between the enhancer and the upstream DNA sequences block enhancement. For example, the DNA of plasmid pBR322 contains multiple enhancer-stop sequences [J. DeVilliers and W. Schaffner (1983), Transcriptional enhancers from papovaviruses as components of eukaryotic expression vectors, in Techniques in the Life Sciences, Vol 5., Nucleic Acid Biochemistry, (R.A. Flavell, ed.), Elserviers Sci. Publ. Ltd., Ireland, pp. 1-20; E. Schreiber et al., Experientia 42, 652-3 (1986)].

## Object of the invention

It is an object of the invention to provide novel hybrid vectors for use in mammalian cells comprising specific arrangements of transcription signals controlling both the expression of a DNA segment coding for a heterologous protein and selection genes assuring that a high proportion of transfected eukaryotic cells stably express the heterologous gene even when said DNA is applied to said eukaryotic cells as circular DNA. It is a further object of the invention to provide eukaryotic host cells transformed with said hybrid vectors and a process for the production of heterologous proteins using said transformed eukaryotic host cells.

## Detailed description of invention

The DNA expression vectors used for eukaryotic cells are generally assembled from preexisting or synthetic DNA in vitro, subsequently transferred and amplified in bacteria usually as plasmids with E.coli as host. At this stage it is verified by restriction digests and/or DNA sequencing whether the DNA has the intended structure. Subsequently, the DNA is introduced into its final host cell, in which the vector will express the gene construct upon which the host cell will produce the desired protein.

## 1. Expression vectors

The invention concerns a recombinant DNA molecule such as a hybrid vector including a circular hybrid vector, comprising a series of functional elements in the following order: (a) an enhancer/promoter unit linked operatively to (b) a DNA segment which can be transcribed into a mRNA having coding capacity for a desired protein product and comprising signals for polyadenylation and for efficient transportation to the cytoplasm, followed by ($c_1$) a promoter unit lacking a functional enhancer, but capable of being stimulated by the enhancer located in (a) due to the absence of an enhancer-stop DNA sequence between (a) and ($c_1$), in turn followed by ($c_2$) a DNA segment which can be transcribed into the mRNA of a selection marker gene

The invention makes use of the finding that transcriptional enhancers can activate more than one set of upstream elements, provided that the enhancer is physically linked to its responding upstream elements. In genetic terms, enhancing is a "cis" effect. Typically, in the expression vector a strong enhancer/promoter combination is placed upstream from the coding portion of the production gene, which is construed in such a way that the transcript will be equipped with signals for mRNA processing (e.g. splicing) and polyadenylation.

Enhancer/promoter units which are suitable to direct transcription of the protein coding region ("production gene") in the vectors according to the invention include, inter alia, the promoter-enhancer of the actin genes, murine or human metallothionein I and II genes, other cellular genes, the enhancer-promoter region of the mouse or human cytomegalovirus major immediate-early gene, the human immunoglobulin enhancer-promoter region, the early enhancer-promoters of papovaviruses, such as Simian virus 40 (SV40), polyomavirus or the various papillomaviruses, or enhancers of the herpes virus genes. Enhancer/promoter units can also be constructed from elements derived from more than one natural gene. Suitable combinations for vectors according to the invention include, inter alia, the SV40 enhancer with the promoter region from the human alpha-globin gene, a metallothionein or cytomegalovirus major immediate-early enhancer with the promoter region from SV40.

A DNA segment coding for a desired (heterologous) protein is especially a DNA (gene) coding for one out of a wide variety of proteins, including glycosylated proteins, in particular of higher eukaryotic, especially mammalian, such as animal or especially human origin, such as enzymes which can be used, for example, for the production of nutrients and for performing enzymatic reactions in chemistry, or polypeptides which are useful and valuable for the treatment of human and animal diseases or for the diagnosis and prevention thereof, for example serum proteins, polypeptide hormones, hormone receptors, lymphokines, interferons, enzymes, enzyme inhibitors, analgesic polypeptides, glycoproteins, immunoglobulins, immunoglobulin-binding proteins, antigenic proteins, vaccine proteins, plasminogen activators or a precursor thereof, such as a corresponding pro- and pre-pro-protein.

Example of such proteins are insulin, growth factors, such as epidermal, insulin-like, mast cell, nerve or transforming growth factor, growth hormones, such as human or bovine growth hormone, interleukin, such as interleukin-1 or -2, human macrophage migration inhibitory factor (MIF) or MIF related polypeptides, interferons, such as human interferon-α, for example interferon-αA, αB, αD or αF, β-interferon, γ-interferon or a hybrid interferon, for example an αA-αD- or an αB-αD-hybrid interferon, hepatitis virus antigens, such as hepatitis B virus surface or core antigen or hepatitis A virus antigen, plasminogen activators, such as tissue plasminogen activator or urokinase, hybrid plasminogen activators, tumour necrosis factor, somatostatin, renin, β-endorphin, immunoglobins, such as the light and/or heavy chains of immunoglobulin D, E or G, immunoglobulin binding factors, such as immunoglobulin E (IgE) binding factor, receptors, such as receptors for viruses, growth factors, lymphokines, interferons, hormones or immunoglobulins, especially immunoglobulin E (IgE) receptor, calcitonin, human calcitonin-related peptide, blood clotting factors, such as factor IX or VIIIc, eglin, such as eglin C, hirudin, desulphatohirudin, or human superoxide dismutase.

Preferably, the hybrid vectors according to the invention contain the 3' untranslated region of a eukaryotic gene comprising signals for proper transcription termination and polyadenylation, such as,

for example, the 3' part of the rabbit β-globin gene, human β-actin
gene or SV40 early gene. If the coding region for the desired
protein is derived from a eukaryotic genomic DNA it usually contains
signals to allow splicing imbedded in the coding sequence. If this
is not the case, for example, because the chosen coding sequence is
derived from a cDNA or from a prokaryotic organism as is the case
for many of the selection markers, it is advantageous to provide
such RNA processing signals in the 5' or 3' untranslated transcribed
regions of the vector.

Suitable splicing signals for vectors according to the invention can
be provided by synthetic DNA [cf. for example G. Rautmann et al.,
EMBO-J. $\underline{3}$, 2021-2028 (1984)], or derived from natural genes, such as
the splicing signals from the 5' intron of the human beta-actin
gene, the 3' intron of beta-globin, the introns of the SV40 early
gene or a combination of signals from different genes, such as a
combination of the first donor site of the adenovirus major late
transcript and the acceptor site of an immunoglobulin gene
[R.J. Kaufman et al., J.Mol.Biol. $\underline{159}$, 601-621 (1982)].

Downstream from the polyadenylation signal contained in functional
element (b) is placed the promoter $(c_1)$ of the marker gene so that
the transcription of the selection gene is in the same direction as
that of the production gene. The distance between the 3'end of the
protein coding region of element (b) and the promoter of the marker
gene is not critical. It can be a few nucleotides to several
thousands provided that care is taken that the DNA sequence in
between the enhancer of functional element (a) and the promoter of
the marker gene does not interfere with the enhancer's desired
stimulatory effect on the promoter of the marker gene. Such enhancer-stop sequences occur frequently in prokaryotic DNA, such as
pBR322, M13, phage lambda or Tn5 DNA, which therefore should be
avoided in this position, but are extremely rare in eukaryotic DNA,
which thus is preferred. As promoter for the selection marker gene
can either be chosen a suitable natural promoter which can be shown
to be dependent on an heterologous promoter in the preferred host cell, for example the adenovirus major late promoter in many cell lines [J.R. Kaufman et al., J.Mol.Biol., 159, 601-621 (1982); F. Asselbergs et al., J. Mol. Biol. 189, 401 (1986)], or a functional promoter which was rendered enhancer-less by deliberate deletion of the enhancer DNA naturally associated with it. Preferred examples of the latter are a fragment of the Herpes virus thymidine kinase promoter [EcoRI-BglI, nucleotides 121-252, S. McKnight, Nucl. Acids Res. 8, 5949 (1980)] and the SV40 early enhancer/promoter minus the so-called 72 bp repeats [L.A. Laimins et al., Proc. Natl. Acad. Sci. USA 79, 6453 (1982); F. Weber et al., Cell 36, 983 (1984)]. Also preferred are enhancer-less deletion derivatives of the promoters taken from the same group considered suitable for the production gene (see above) in which case, however, to avoid homologous genetic recombination care is to be taken to minimize or avoid altogether the use of the same DNA sequence in both production and selection marker gene. This consideration is also taken into account when DNA sequences are chosen to provide the selection gene with RNA processing signals, which can be chosen from the same group of genes as indicated for the production gene processing signals. For example, as signal for splicing and polyadenylation can be placed downstream from the 'product' coding sequence the 3' portion of the rabbit beta-globin gene whereas the splicing signals for the selection gene can be provided by an adenovirus/immunoglobulin hybrid intron placed upstream of the coding sequence, which can encode mouse DHFR, and the 3' polyadenylation signal of the selection gene by the early polyadenylation signal from SV40 placed downstream (Kaufman et al., supra).

Behind such a promoter consisting typically of "upstream elements" only is placed the coding sequence ($c_2$) for the selection marker gene suited to the type of host cell used. The marker gene may be of eukaryotic or prokaryotic origin. Preferred are antibiotic resistance genes, such as, for example, the genes conferring resistance against bleomycin [P. Mazodier et al., Nucl.Acids Res. 13, 195-205 (1985); D. Semon et al., Plasmid 17, 46-53 (1987)], hygromycin-B and neomycin-type antibiotics [listed in Brenner, Nature 329, 21 (1987)]. Geneticin, previously known as G418, is the neomycin-type antibiotic most often used for selection of eukaryotic cells. Also preferred are genes that provide cells with novel biosynthetic pathways thus suppressing cellular auxotrophy. Good examples are thymidine kinase (tk) genes (for tk⁻ cells), dihydrofolate reductase (DHFR) genes (for dhfr-cells) and the xanthine-guanine-phosphoribosyltransferase (XGPRT) gene (for mammalian cells cultured in the presence of xanthine, aminopterin and mycophenolic acid). Further marker proteins include enzymes that produce coloured substances from substrates having no or a different colour. Among the preferred selection genes feature prominently those that can be used to induce gene amplification such as the dihydrofolate reductase (DHFR) gene, expecially a variant with a reduced affinity for its inhibitor methotrexate, adenosine deaminase gene, the thymidine kinase gene of herpes simplex virus, and those listed in G.R. Stark et al., "Gene Amplification", in Ann. Rev. Biochem. 53, 447-491 (1984).

Preferably, the vectors of the invention contain an origin of replication and an antibiotic resistance gene, such as amp or tet, for propagation in E.coli. A mammalian origin of replication may be provided either by including in the construction of the vector a eukaryotic origin, such as derived from SV40 or from another viral source, or may be provided by the host cell chromosome upon integration of the vector into the host cell chromosome. The SV40 origin of replication is contained, for example, in the HindIII-SphI fragment of the viral DNA [nucleotides 5171 to 128, origin = position 1; J. Tooze (ed.) DNA Tumor Viruses, Part. 2, 2nd Edition, Cold Spring Harbor Laboratory, Cold Spring Harbor N.Y. 1982].

In order to shield the dual gene construct above from the influence of extraneous enhancers such as located on the host chromosome, it is preferred to have enhancer-stop DNA upstream from the promoter/enhancer of the production gene and downstream of the promoter of the selection gene. If the coding DNA sequence of the selection gene does not already have enhancer-stop activity, it is preferred to provide for the presence of such DNA behind the selection gene.

Preferred source of enhancer-stop DNA is prokaryotic DNA. If no enhancer-stop DNA sequences are present in coding sequences of the selection gene, such as is often the case when a eukaryotic gene like the Herpes virus thymidine kinase gene is used, nor in the 5' and 3' untranslated regions the entire gene can also be inverted so that transcription is in the opposite direction of the production gene.

The dual gene construct described above is preferably propagated as a plasmid or bacteriophage in an intermediate host, usually a bacterium, such as E. coli. If a plasmid-type replicon is preferred the above dual gene construct is combined with a fragment of a bacterial plasmid containing a selection gene and a replication signal active in the bacterial host. Preferably these are taken from a pBR322 derivative, such as pSVOd [P. Mellon et al., Cell 27, 279-288 (1981)] in which the so-called poison sequence which would inhibit the SV40 T antigen-driven replication of the vector, is removed.

Those parts of the recombinant DNA molecules according to the invention which contain an origin of replication and a selection marker gene usually both of bacterial origin are referred to as "vector DNA".

The recombinant DNA molecule according to the invention are produced by linking said functional elements (a) to (d) to a vector DNA such that they are arranged in the predetermined order.

Various techniques may be used to link DNA segments in vitro. Blunt ends (fully base-paired DNA duplexes) produced by certain restriction endonucleases may be directly ligated with T4 DNA ligase. More usually, DNA segments are linked through their single-stranded cohesive ends and covalently closed by a DNA ligase, e.g. T4 DNA ligase. Such single-stranded "cohesive termini" may be formed by cleaving DNA with another class of endonucleases which produce staggered ends (the two strands of the DNA duplex are cleaved at different points at a distance of a few nucleotides). Single strands can also be formed by the addition of nucleotides to blunt ends or staggered ends using terminal transferase ("homopolymeric tailing") or by simply chewing back one strand of a blunt-ended DNA segment with a suitable exonuclease, such as λ exonuclease. A further approach to the production of staggered ends consists in ligating to the blunt-ended DNA segment a chemically synthesized linker DNA which contains a recognition site for a staggered-end forming endonuclease and digesting the resulting DNA with the respective endonuclease. The components of the hybrid vectors according to the invention are linked together in a predetermined order to assure proper function. General techniques used in vector constructions are described in "Molecular Cloning, a laboratory manual" by T. Maniatis et. al. (Cold Spring Harbor Lab. Cold Spring Harbor N.Y., 1982).

2. Eukaryotic hosts transformed with the hybrid vectors

Another aspect of the present invention involves eukaryotic host cells transformed with the recombinant DNA molecules, especially hybrid vectors, according to the invention and to the process for the preparation thereof. Suitable final hosts for the expression vectors according to the invention are those in which enhancer-controlled gene expression operates. This includes the cells of the higher eukaryotes, such as fungi, plants and animals.

Examples of suitable eukaryotic hosts are green plants, fungi, established mammalian, such as human or animal cell lines, e.g. myeloma cells, human embryonic lung fibroblasts L-132, LTK⁻ cells, human Bowes melanoma cells, HeLa cells, SV-40 virus-transformed kidney cells of African green monkey, such as COS cells, or chinese hamster ovary (CHO) cells and variants thereof. Mutants of trans-formed host organisms include especially mutants which are poor in protein degrading proteases, give higher yields in the heterologous protein, grow in protein-poor media or which are deficient in expression of the selection gene of the vector.

- 11 -

The process for the production of a transformed host comprises transforming a host with the recombinant DNA molecules according to the invention.

The transformation of eukaryotic host cells, such as mammalian host cells, is accomplished by methods known in the art. The introduction of hybrid vectors into mammalian cells is done by transfection in the presence of helper compounds, e.g. diethylaminoethyldextran, dimethyl sulfoxide, glycerol, polyethylene glycol, DEAE-dextran, polyornithine, chloroquin, sodium butyrate or the like, to facilitate DNA uptake, or as co-precipitates of vector DNA and calcium phosphate. Further suitable methods include direct microinjection of vector DNA into the cell nucleus and electroporation, i.e. introduction of DNA by a short electric pulse increasing the permeability of cell membranes. During transformation, the cells are usually cultured under non-selective conditions. The subsequent selection of transfected cells can be done using a selection marker which was either covalently integrated into the expression vector or added as a separate entity during transfection. The selection markers include genes which confer resistance to antibiotics or genes which complement a genetic lesion of the host cell (supra).

One preferred selection system makes use of cells lacking dihydrofolate reductase (DHFR$^-$), e.g. CHO cells, which absolutely require thymidine, glycine and purines for growth unless an exogenous DHFR gene is supplied. On introduction of a hybrid vector containing a DHFR gene into suitable DHFR$^-$ cells, e.g. CHO cells, transformed cells are selected by increasing the concentration of the antifolate drug methotrexate in the medium.

Another preferred selection is a method wherein suitable mammalian cells, e.g. CHO cells, are treated with co-precipitates of vector DNA containing a gene coding for antibiotics resistance, e.g. resistance to G-418, and calcium phosphate. The transformed cells

- 12 -

are selected by culturing in the presence of the corresponding antibiotics, e.g. G-418, and/or by screening for expression of the desired protein.

### 3. Novel aspects of the invention

In general, only part of the cells take up the DNA and in only a small percentage of the transfected cells the DNA become stably integrated into the chromosome of the host cell. Therefore, shortly after transfection culture conditions of the cells are changed such that the new culture conditions only allow propagation of cells expressing the marker gene. After a suitable growth period, typically 2-4 weeks for most tissue culture cell lines, colonies of tranformed cells become apparent. Cells from a number of such colonies are then cultured further in separate culture vessels and have to be tested for expression of the production gene because generally only part of the colonies express the production gene. In contrast, at this stage it is unexpectedly observed that, if hybrid vectors according to the invention are used, virtually all colonies express the production gene. In fact, for most purposes it is no longer necessary to select and test individual colonies, as a culture of mixed transformants would produce equally well as cells derived from a typical transformed colony. Apart from obviating an elaborate series of tests, this procedure reaches the production phase faster.

### 4. Further improvement of the initial transformant

The transformed host organisms according to the invention can be improved in the production of heterologous proteins by mutation and selection applying methods known in the art. The mutation can be effected, for example, by U.V. irradiation or suitable chemical agents. Especially preferred is a method whereby cells are selected which have an increased expression of the marker gene due to an increase in copy number of the vector DNA, such as occurs when amplifiable genes, for example those listed in Stark et al., ["Gene amplification", in Ann. Rev. Biochem. 53, 447-491, (1984)] are used as selection gene. For example, if the selection gene is an enzyme limiting the synthesis of a cell component this can be achieved by gradually increasing the concentration of a competitive inhibitor (in the case of DHFR the substrate analog methotrexate). Alternatively, if the selection gene encodes an enzyme that modifies the antibiotic, the increased selective pressure needed for gene amplification can be achieved by raising the antibiotic concentration. Also preferred is the production of protease-deficient mutants, in order to avoid proteolytic degradation of the produced protein.

5. Cultivation of transformed host cells

The invention concerns furthermore a method for the production of a desired protein comprising culturing under appropriate nutrient conditions a transformed eukaryotic host comprising a recombinant DNA molecule according to the invention and isolating said protein.

The transformed host cells are cultured by methods known in the art in a liquid medium containing assimilable sources of carbon, nitrogen and inorganic salts. The culture conditions, such as temperature, pH value of the medium and fermentation time, are chosen such that a maximum titre of the heterologous protein is obtained.

Mammalian cells are grown under tissue culture conditions using commercially available media optionally supplemented with growth-promoting substances and/or mammal sera. The cells are grown either attached to a solid support, e.g. a microcarrier or porous glass fibres, or free-floating in appropriate culture vessels. The culture medium is preferably selected in such a way that selection pressure is exerted and only those cells survive which still contain the hybrid vector DNA including the genetic marker. Thus, for example, an antibiotic is added to the medium when the hybrid vector includes the corresponding antibiotic resistance gene.

When the cell density has reached a sufficient value culturing is interrupted and the protein isolated. As the protein is usually secreted into the medium, alternatively, the medium containing the product can be separated from the cells which can be provided with fresh medium and used for continuous production. The resulting mixture is enriched for the desired protein by conventional means, such as removal of most of the non-proteinaceous material by treatment with polyethyleneimine, precipitation of the proteins using ammonium sulphate, gel electrophoresis, dialysis, chromatography, for example, ion exchange chromatography, size-exclusion chromatography, HPLC or reverse phase HPLC, molcular sizing on a suitable Sephadex® column, or the like. The final purification of the pre-purified product is achieved, for example, by means of affinity chromatography, for example antibody affinity chromatography, especially monoclonal antibody affinity chromatography using monoclonal antibodies fixed on an insoluble matrix by methods known in the art.

The invention concerns especially the recombinant DNA molecules, transformed hosts and the methods for the preparation thereof and the methods for producing a desired protein by means of said transformed hosts, as described in the Examples.

Brief description of the drawings

In the following experimental part various embodiments of the present invention are described with reference to the accompanying drawings in which:

Fig. 1 schematically depicts the construction of plasmid pDO2.

Fig. 2 schematically illustrates the construction of plasmid pDO10 containing the t-PA cDNA combined with a beta globin fragment.

Fig. 3 schematically illustrates the construction of plasmid pCGA26 containing the t-PA cDNA under control of the MCMV IE promoter and a beta globin fragment.

Fig. 4 schematically illustrates the construction of t-PA expression plasmid pCGA28 and of universal expression plasmid pCGA44, both plasmids including the neomycin resistance gene.

Fig. 5 schematically illustrates the construction of t-PA expression plasmid pCGA48 including the neomycin resistance gene and the DHFR gene.

Fig. 6 schematically illustrates the construction of plasmid pCGA31, capable of expressing human t-PA under the SV40 early promoter and a derivative, pCGA31-dhfr, which in addition expresses mouse DHFR.

Fig. 7 schematically illustrates the construction of plasmid pCGA39, capable of expressing SV40 tumor antigens under control of the SV40 early promoter with a mouse metallothionein enhancer.

Fig. 8 schematically illustrates the construction of plasmid pCGA46, capable of expressing human t-PA under control of the SV40 early promoter with a mouse metallothionein enhancer and a derivative, pCGA46-dhfr, which in addition expresses mouse DHFR.

Fig. 9 schematically illustrates the structure of the human IgE cDNA plasmid and construction of plasmid pCAL3.

Fig. 10 schematically illustrates the construction of plasmid pSVG-ER, capable of expressing human IgE receptor under control of the SV40 early promoter and a derivative, pSVG-ER-dhfr, which in addition expresses mouse DHFR.

Fig. 11 schematically illustrates the construction of plasmid pSVG-BF capable of expressing human IgE-binding factor under the SV40 early promoter and a derivative, pSVG-BF-dhfr, which in addition expresses mouse DHFR.

Fig. 12 schematically illustrates the construction of plasmid pMG-ER, capable of expressing human IgE receptor under control of the MCMV major immediate-early promoter and a derivative, pMG-ER-dhfr, which in addition expresses mouse DHFR.

Fig. 13 schematically illustrates the construction of plasmid pMG-BF capable of expressing human IgE-binding factor under the MMTV major immediate-early promoter and a derivative, pMG-BF-dhfr, which in addition expresses mouse DHFR.

Fig. 14 schematically illustrates the construction of plasmids pSVMTG-ER and pSVMTG-BF capable of expressing human IgE receptor and IgE-binding factor respectively under control the SV40 early promoter with a mouse metallothionein enhancer and derivatives, pSVMTG-ER-dhfr and pSVMTG-BF-dhfr, which in addition express mouse DHFR.

Fig. 15 schematically depicts the structure of plasmids pNE, an expression vector containing an SV40 early promoter lacking enhancer activity, and pSV2neo, pSV2gpt and pKC214, donor plasmids for the coding sequences of selection genes.

Fig. 16 schematically depicts the structure of plasmids pNE-neo, pNE-gpt, pNE-hph and pMK, plasmids from which selection genes without enhancer activity can be excised conveniently.

Fig. 17 schematically depicts the structure of plasmids pSVG-tPA-neo, pSVG-tPA-gpt, pSVG-tPA-hph and pSVG-tPA-tk, t-PA-expression plasmids containing different selection genes.

- 17 -

Fig. 18 schematically depicts the structure of plasmids pMG-BF-neo, pMG-BF-gpt, pMG-BF-hph and pMG-BF-tk, IgE-binding factor expression plasmids containing different selection genes.

Fig. 19 schematically illustrates the construction of neo$^R$ plasmid pBR3a, capable of expressing human urokinase-type plasminogen activator (u-PA) and a derivative, pBR3a-dhfr, which in addition expresses mouse DHFR.

Symbols used in the accompanying figures have the following meanings:

| | |
|---|---|
| AMP, Amp$^R$ | ampicillin resistance gene (beta-lactamase) |
| TET, Tet$^R$ | tetracyclin resistance gene |
| NEO, neo$^R$ | Tn5 neomycin phosphotransferase |
| TN5PR | bacterial promoter of transposon TN5 |
| HPH | hygromycin phosphotransferase |
| pBRori | origin of replication of plasmid pBR322 |
| POIS | 'poison-sequence', pBR322 sequence which is inhibitory to SV40 replication |
| SV40ori | origin of replication of SV40, coincides with early and late promoters. |
| SV40enh, SV40E | 72 bp enhancer, part of SV40 early promoter |
| HCMVE | enhancer of human cytomegalovirus (HCMV) major immediate early gene |
| MCMVP | promoter/mRNA start site of mouse cytomegalovirus (MCMV) major immediate early gene |
| RSV | Rous sarcoma virus LTR (promoter) |
| CAP | position of 5' m7Gp 'cap' of eukaryotic mRNA |
| polyA | polyadenylation site of mRNA |
| SPLD | splice donor site, 5' end of intron |
| SPLA | splice acceptor site, 3' end of intron |
| BAP | bacterial alkaline phosphatase |
| CIP | calf intestinal phosphatase |

0 271 003

- 18 -

| (BamH1/Bgl2) | Sau3a site resulting from coligating a BamHI and a BglII site |
| x < y | restriction enzyme site x located clockwise from y |
| p | promoter |
| G/C-tail | homopolymer of G and C residues |

## Experimental Part

## Example 1: Construction of expression vector pCGA48

## A) Conversion of the HgiAI site in t-PA cDNA to a HindIII site

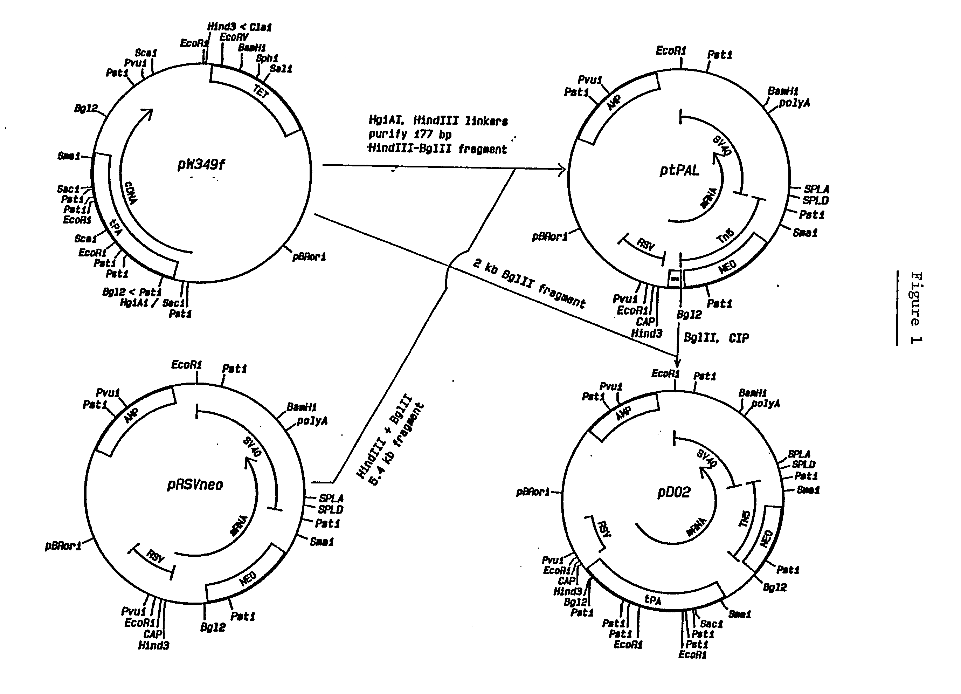

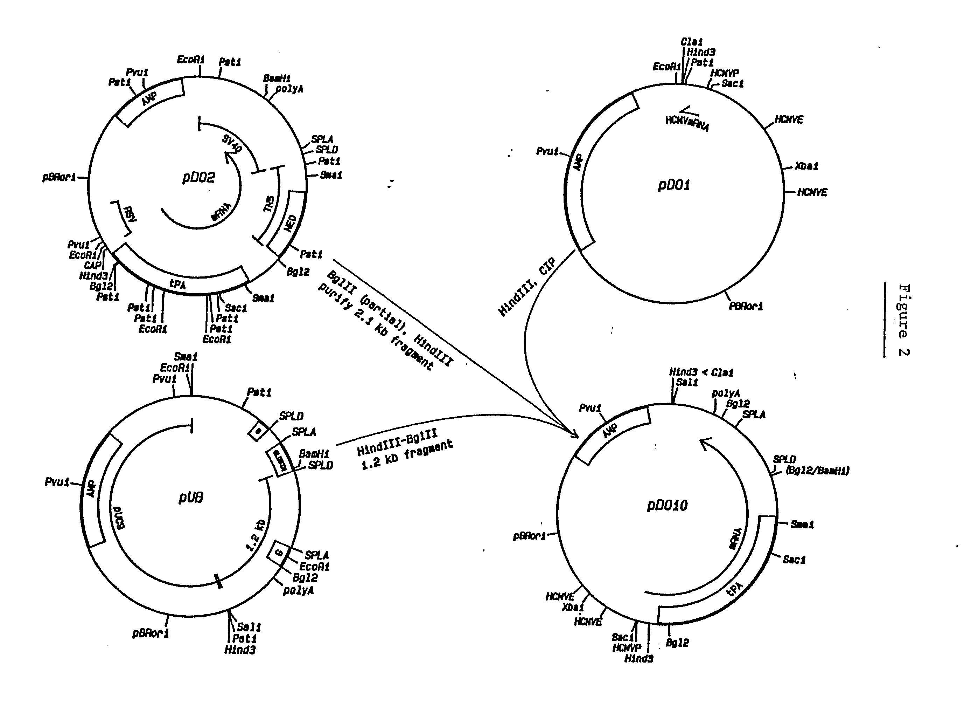

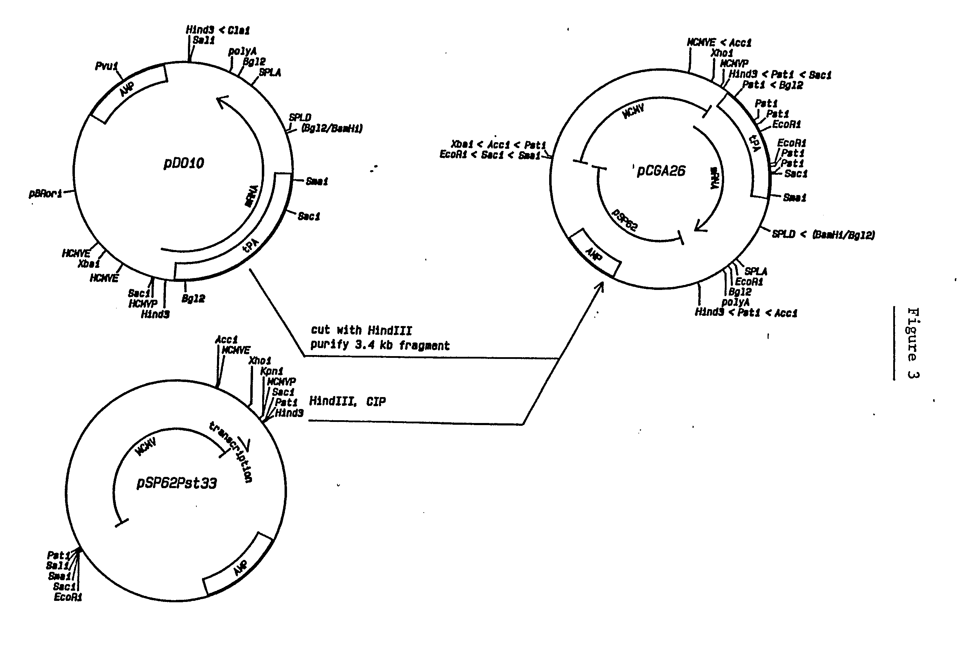

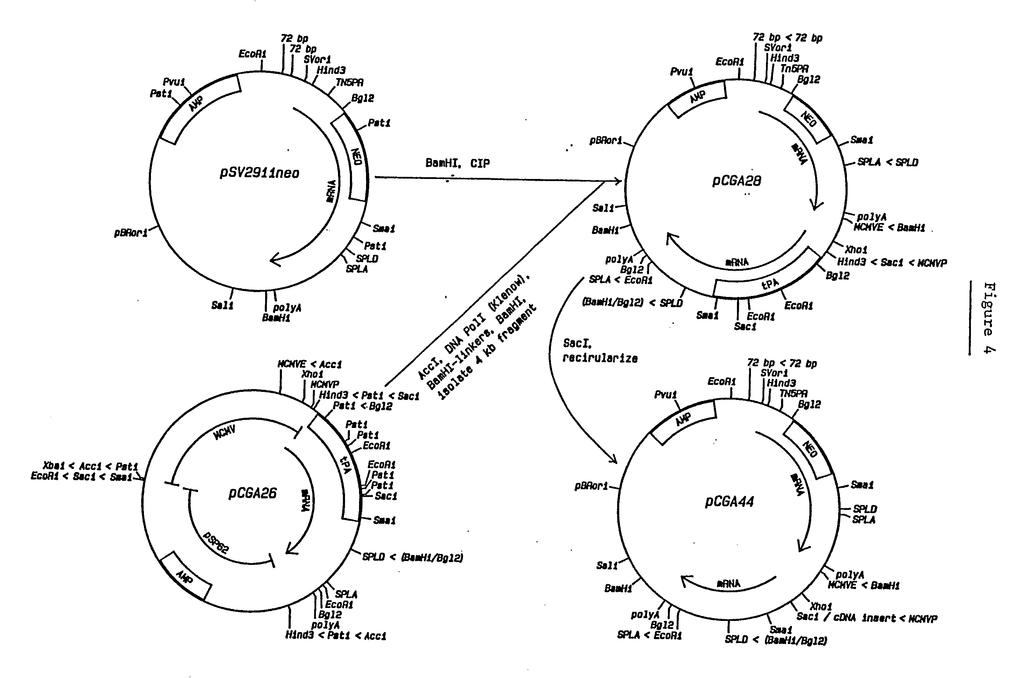

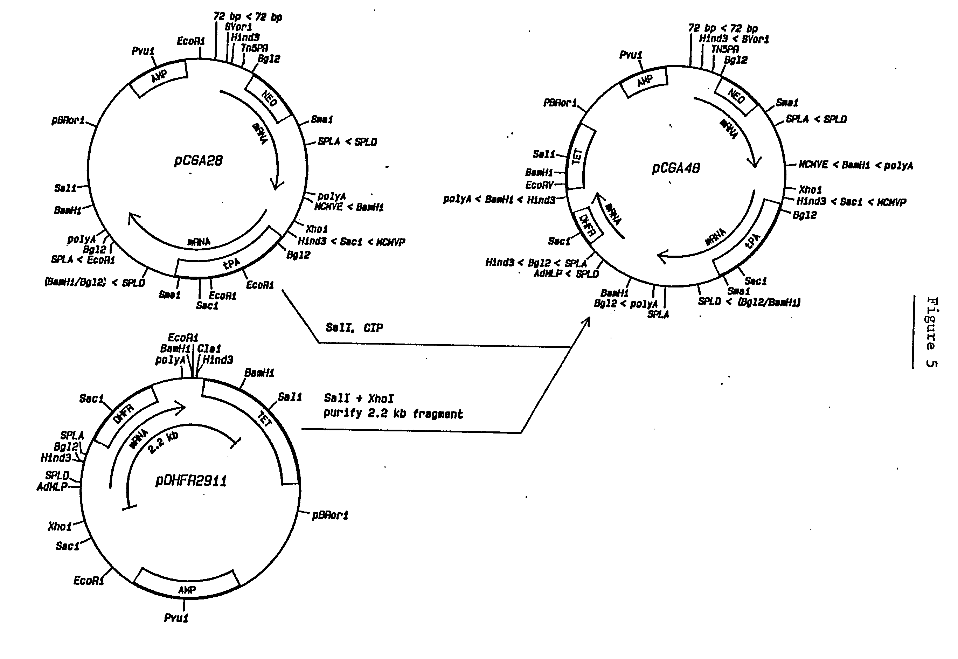

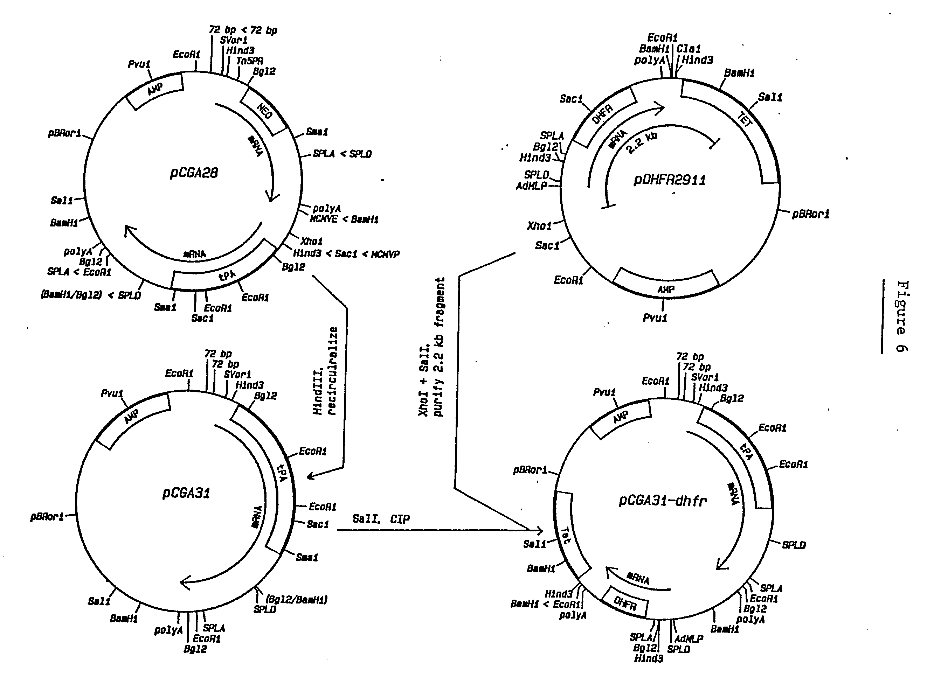

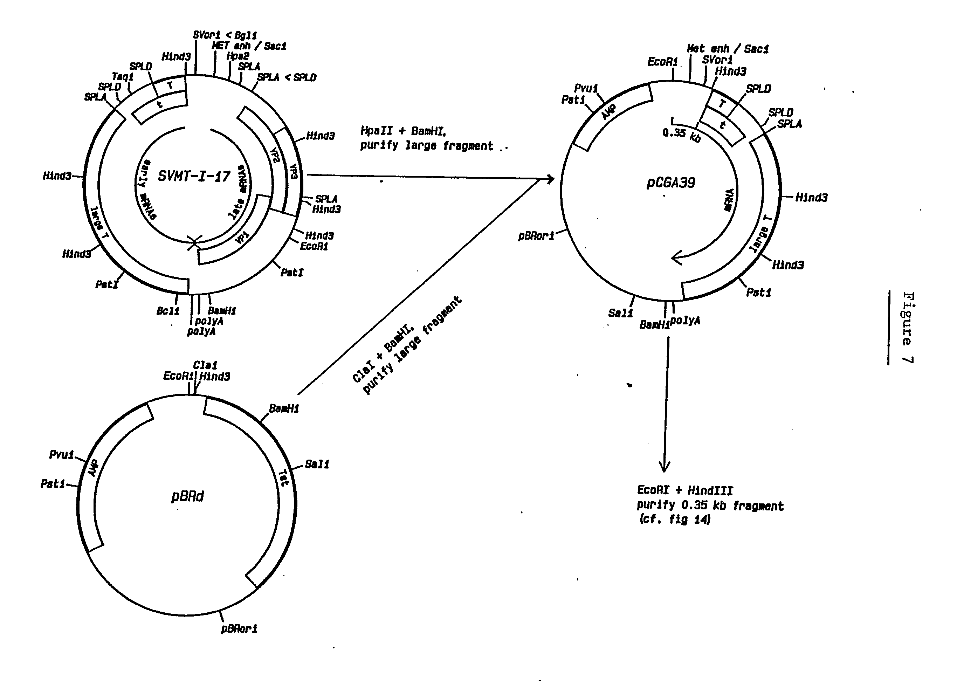

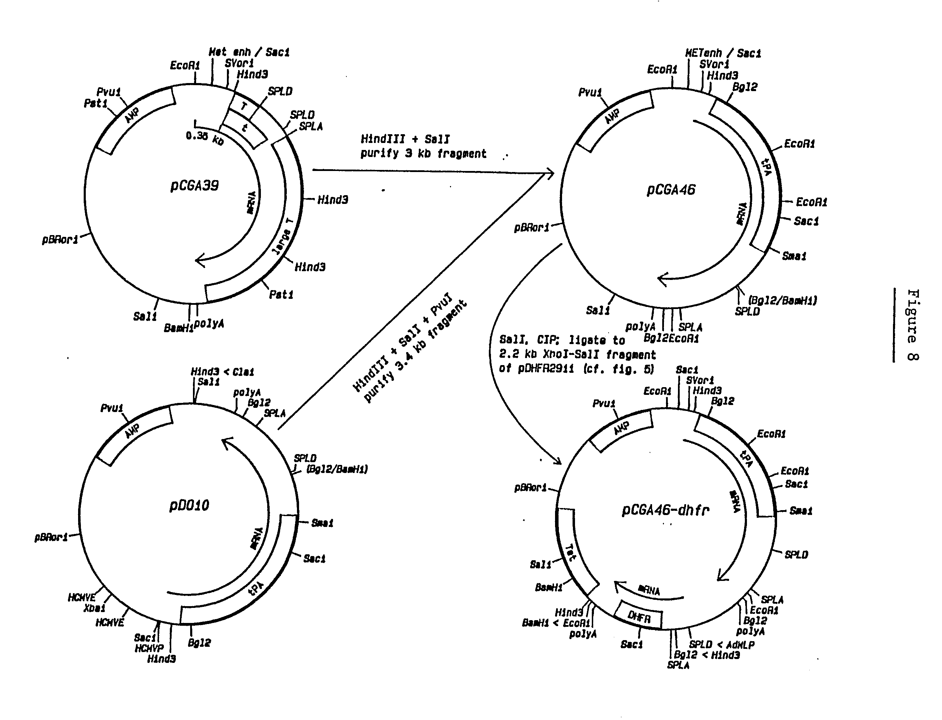

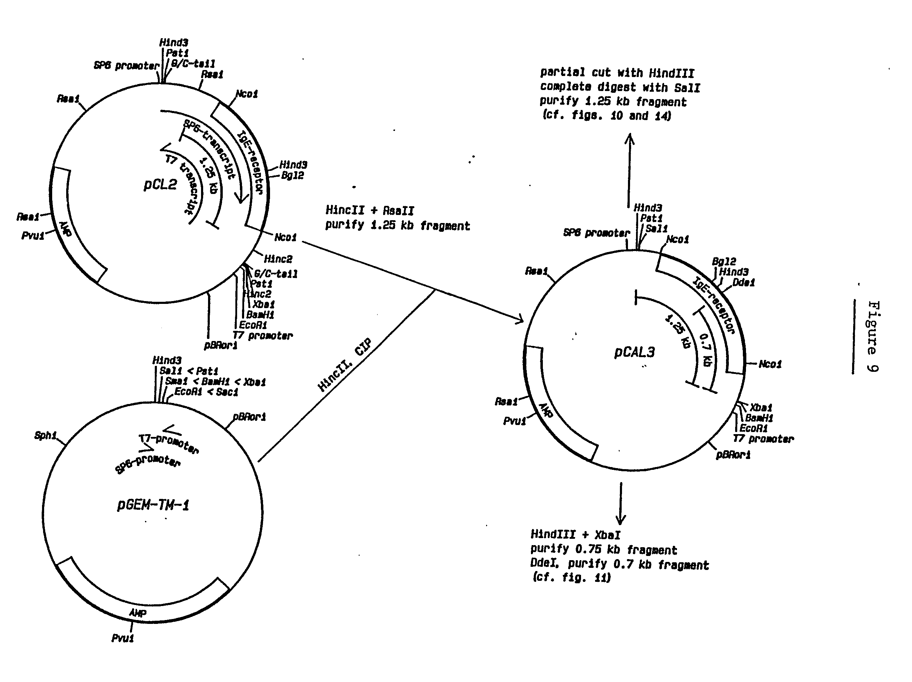

This is achieved in five steps (fig. 1).

Plasmid pW349F (7.0 kb; European Patent Application No. 143,081) is partially cleaved with the restriction enzyme HgiAI by incubation of 20 µg/ml DNA for 1 h at 37°C with 12 U/ml of the enzyme in the buffer recommended by the manufacturer (Bethesda Research Laboratories) except that it is supplemented with 10 µg/ml ethidium bromide to suppress secondary cutting of the plasmid. Linearized plasmid DNA is then applied to a 0.8 % agarose gel in TBE buffer (TBE: 89 mM Tris-borate pH 8.9 containing 1 mM EDTA), electrophoretically eluted in the same buffer, twice extracted with phenol, twice with chloroform and finally precipitated with alcohol at -20°C after addition of 0.1 vol. of 3 M sodium acetate ph 5.2. Pelleted DNA is dissolved at 0.2 mg/ml in TE (TE: 10 mM Tris-HCl pH 7.2 with 0.1 mM EDTA).

63 µl of linearized DNA is then incubated for 30 min. at 37°C with 15 U of T4 DNA polymerase in ligase buffer [33 mM Tris-acetate (pH 7.9), 66 mM potasium acetate, 10 mM magnesium acetate, 0.5 mM dithiothreitol and 0.1 mg/ml bovine serum albumin] followed by heating 10 min. at 60°C to inactivate the enzyme. The purpose of this incubation is to use the exonucleolytic activity of the T4 polymerase to remove the protruding four nucleotides left after digestion with HgiAI to obtain blunt-ended DNA molecules.

In order to ligate HindIII linkers (CAAGCTTG) to the blunt-ended DNA
6 µl (300 ng) kinased linkers are added to the above solution with
4 µl 10 mM ATP and 3 µl T4 DNA ligase (New England Biolabs,
400 u/µl) followed by a 16 h incubation at 16°C. The ligation is
terminated by heating the mixture 10 min. at 68°C, after which the
DNA is digested with HindIII and BglII, i.e. 15 µl (135 U) HindIII
is added with 1.5 µl 4M NaCl, 0.2 µl 1M $MgCl_2$ and 11 µl 1 mg/ml
bovine serum albumin, incubated at 37°C for 1 h followed by addition
of 40 U BglII followed by another 1 h incubation at 37°C. The
resulting 177 base pair fragment is purified on a 6 % polyacrylamide
gel run in TBE, eluted in TNE (TNE: 10 mM Tris-HCl pH 8.8 containing
100 mM NaCl and 1 mM EDTA), absorbed to DEAE cellulose (Whatman
DE52), eluted with 1 M NaCl in TNE, diluted with 4 volumes of water,
precipitated at -20°C after addition of 2.5 volumes of ethanol and
finally dissolved in 17 µl TE (TE: 10 mM Tris-HCl pH 8.0 containing
1 mM EDTA).

Plasmid pRSVneo (5.7 kb; ATCC 37198) is a derivative of plasmid
pSV2neo [P.J. Southern and P. Berg, J. Mol. Appl. Genet. 1, 327-341
(1982)] in which the SV40 -derived PvuII-HindIII fragment has been
replaced with a PvuII-HindIII fragment containing the LTR promoter
from Rous sarcoma virus in the same manner as pRSVcat was constructed from pSV2cat [C.M. Gorman et al., Proc. Natl. Acad. Sci.
USA 79, 6777-6781 (1982)]. 5 µg of this plasmid is cut in a 50 µl
volume with 24 U BglII according to the manufacturer's instructions.
After a 1 h incubation at 37°C 40 U HindIII are added and the
incubation continued for 1.5 hour after which the large 5.4 kb
fragment is purified as described above.

17 µl of the purified 177 bp fragment are ligated for 18 hours at
16°C to 2 µl (20 ng) of the pRSVneo fragment using 0.25 µl (100 U)
T4 ligase in a total volume 22 µl ligase buffer, after which the
plasmid DNA is used to transform E.coli according to D. Hanahan [J.
Mol. Biol. 166, 557-580 (1983)]. From the resultant ampicillin-
resistant strains one is selected containing a plasmid designated ptPAL (5.6 kb) with the 177 bp HindIII-BglII fragment as evidenced by restriction analysis . 0.1 µg of this plasmid is cut in 60 µl with 16 U BglII as recommended by the manufacturer for 1.5 h at 37°C. To this solution is then added 20 U calf intestinal alkaline phosphatase (Boehringer Mannheim) and the incubation continued for 30 min. after which the DNA is extracted twice with phenol, twice with chloroform and precipitated after adding 0.1 volume 3.0 M sodium acetate pH 5.2 and 0.6 volume of isopropanol, dissolved in TE, further purified by agarose gel electrophoresis as described above, twice extracted with phenol, twice with chloroform, precipitated at -20°C after addition of 2.5 volumes ethanol and 0.1 vol. 3 M sodium acetate pH 5.2 and finally dissolved in 30 µl TE. The 2.1 kb t-PA BglII fragment is then cut out of 5 µg pW349F in a 25 µl reaction using 20 U BglII for 2 h at 37°C, purified on a 0.8 % agarose gel, electrophoretically eluted as described above, twice extracted with phenol, twice with chloroform, precipitated at -20°C after addition of 2.5 volumes ethanol and 0.1 vol. 3 M sodium acetate pH 5.2 and dissolved at a concentration of 8 ng/µl in TE. 1 µl of the t-PA fragment is then ligated in a 10 µl reaction to 7.5 ng BglII cut vector DNA using 100 U T4 ligase (Biolabs) for 17 h at 16°C and subsequently transformed into E.coli. One of the resultant clones, designated pDO2 (7.6 kb), contains the t-PA BglII fragment inserted in such a way that the plasmid contains a continuous open reading frame for human t-PA.

B) Combination of the t-PA cDNA with the beta-globin fragment

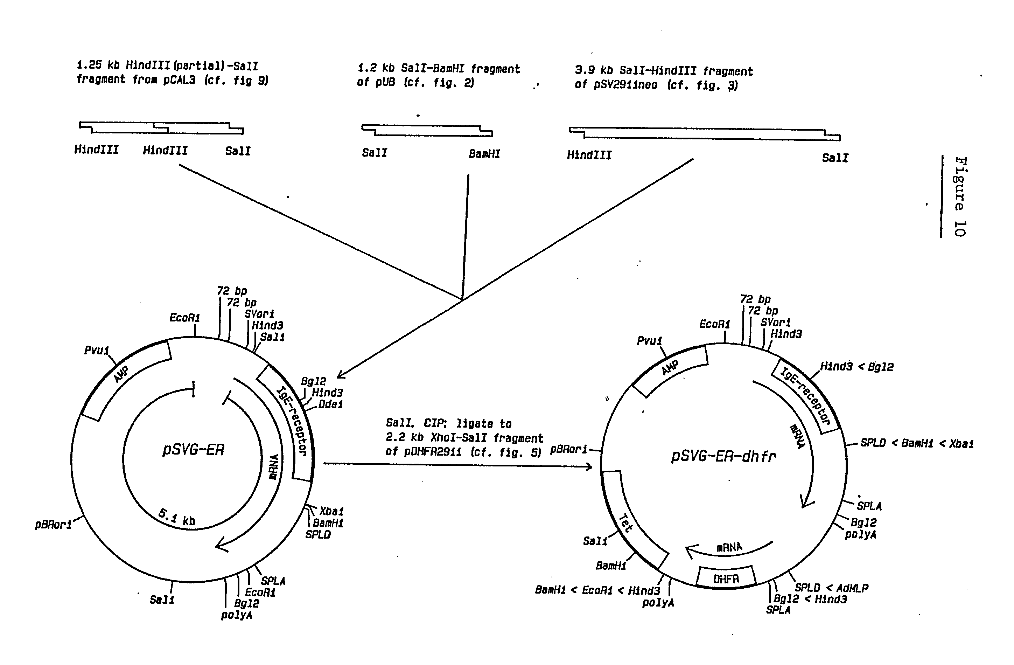

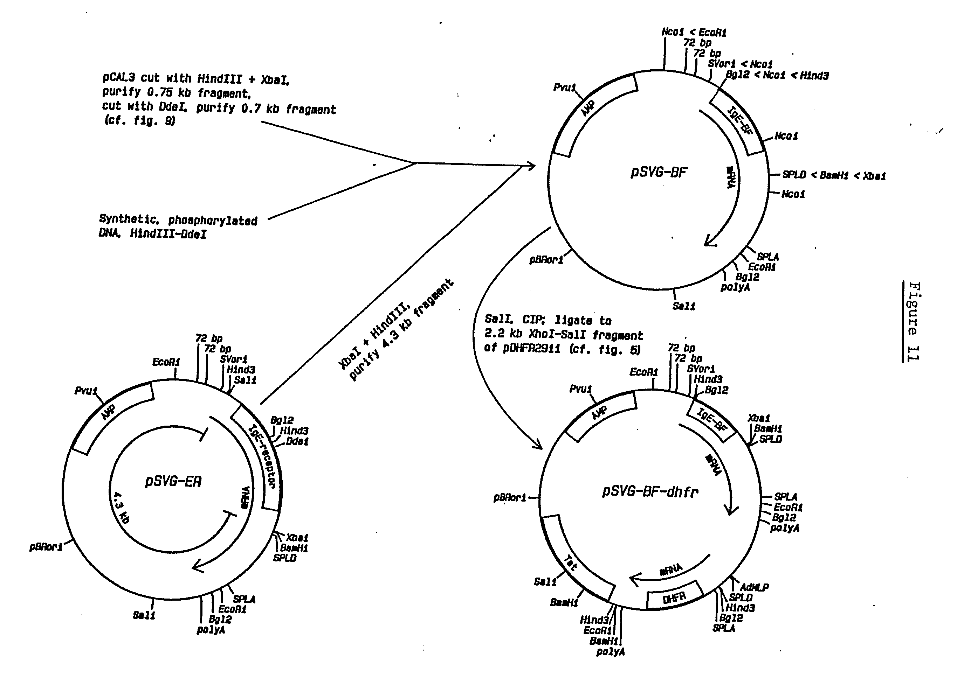

Plasmid pDO10 (6.5 kb; fig. 2) is constructed by coligating three DNA fragments: (i) a 2.1 kb fragment starting with a HindIII site and terminating with a BglII site containing the whole t-PA coding sequence is isolated from an agarose gel on which is loaded 10 µg of pDO2 DNA cut partially with BglII and completely with HindIII. (ii) pUB [4.8 kb; F. Weber et al., Nature 315, 75-77 (1985)] is a plasmid containing the rabbit beta globin gene [A. Van Ooyen et al., Science 206, 337 (1979)] subcloned as a BglII partial digest into the BamHI site of plasmid pUC9 [J. Vieira and J. Messing, Gene 19, 259-268 (1982); ibid. 19, 269-276 (1982)]. From this plasmid a 1.2 kb BamHI-HindIII fragment containing the second intron and the polyadenylation site is excised and purified by agarose gel electrophoresis. (iii) Vector pDO1 (3.2 kb) is built up, in anticlockwise order from the HindIII site (fig. 2) of the HindIII-AccI fragment of pBR322 which includes the origin of replication, a 0.3 kb fragment containing the enhancer of human cytomegalovirus (HCMV) terminating in a synthetic XbaI site followed by a second copy of this enhancer attached to the homologous promoter terminating at a synthetic HindIII site. This vector DNA is cut with HindIII and the 6.3 kb linear plasmid is purified by agarose gel electrophoresis.

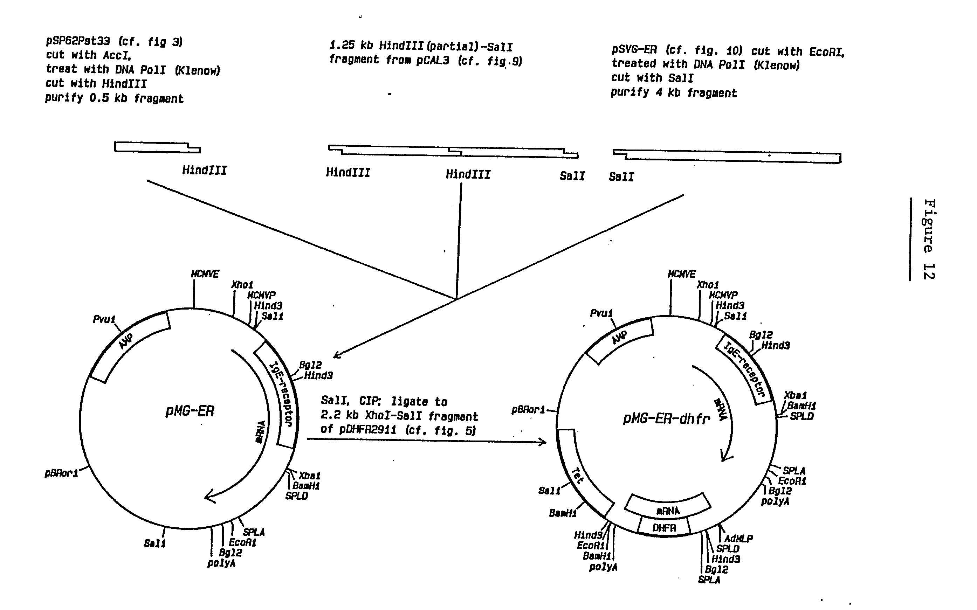

C) Inserting the tPA/globin combination into pSP62Pst33 (see Fig. 3)

pSP62Pst33 (6.2 kb; fig. 3) is a plasmid containing a 2.1 kb PstI fragment of the mouse cytomegalovirus (MCMV) DNA, which includes the viral immediate early (IE) promoter, inserted into the PstI site of plasmid pSP62 (Boehringer Mannheim) as indicated in the figure. Into the HindIII site of pSP62Pst33 is inserted the HindIII fragment from pDO10. A plasmid, pCGA26 (9.6 kb), is selected in which the t-PA coding sequence is inserted such that it can be transcribed in "sense" orientation from the MCMV IE promoter.

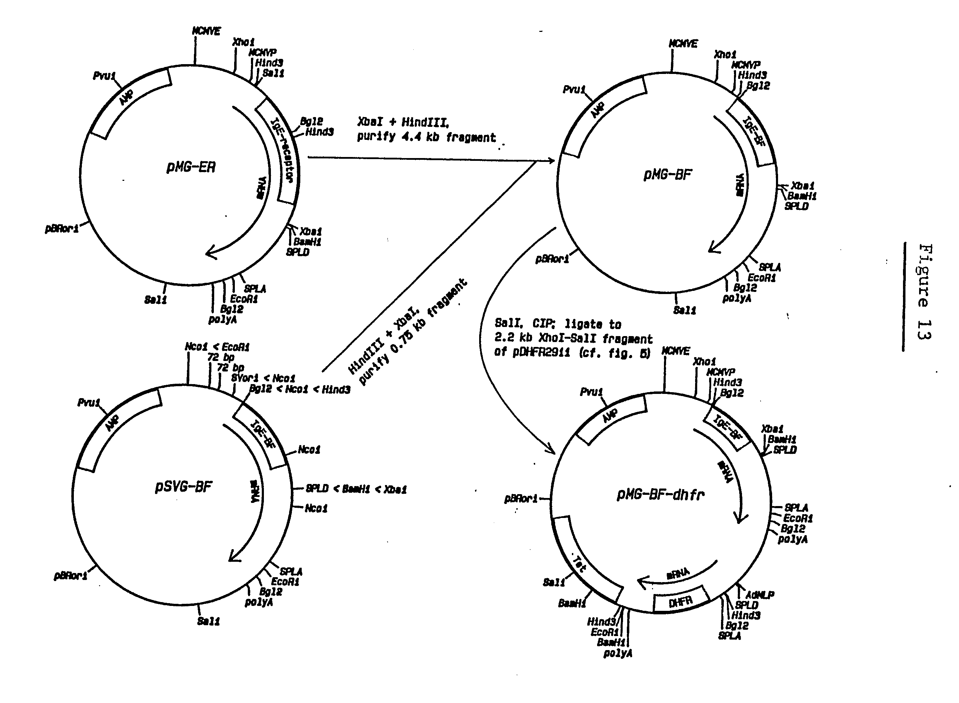

D) Inserting the MCMV/tPA/globin unit into pSV2911neo (see Fig. 4)

Plasmid pSV2911neo [5.7 kb; F. Asselbergs et al., J. Mol. Biol. 189, 401-411 (1986)] contains the neomycin (neo) phosphotransferase gene from transposon TN5 in an SV40 expression cassette (Fig. 4). Thus it confers resistance to neomycin and kanamycin in bacteria and to geneticin (G418) when introduced into mammalian tissue culture cells. pSV2911neo DNA is prepared for cloning by cutting with BamHI, treating with calf intestinal alkaline phosphatase, two extractions with phenol, two with chloroform, precipitation with alcohol and finally dissolved in TE. Plasmid pCGA26 is cut with restriction enzyme AccI, which cuts the sequence GT/ACAC at position 345 in the MCMV enhancer/promoter region [K. Doersch-Haessler et al., Proc. Natl. Acad. Sci. USA 82, 8325-8329 (1985)] and the sequence GT/CGAC (can also be cut with SalI) behind the globin part. The two base overhangs resulting after cutting are filled in with E.coli (large fragment) DNA polymerase I, the now blunt ends are ligated to BamHI linkers (CGGATCCG) and these cut with BamHI enzyme. The 3.8 kb fragment carrying the MCMV/tPA/ globin unit now with BamHI ends is purified via an agarose gel and then ligated to the pSV2911neo DNA prepared as described above to yield expression plasmid pCGA28.

A general cDNA-expression vector, pCGA44 (Fig. 4), is prepared by cutting pCGA28 with SacI followed by recircularization of the resulting large DNA fragment. Thus, most of the t-PA coding sequences have been removed and another coding sequence can be conveniently inserted at the unique SacI site of pCGA44.

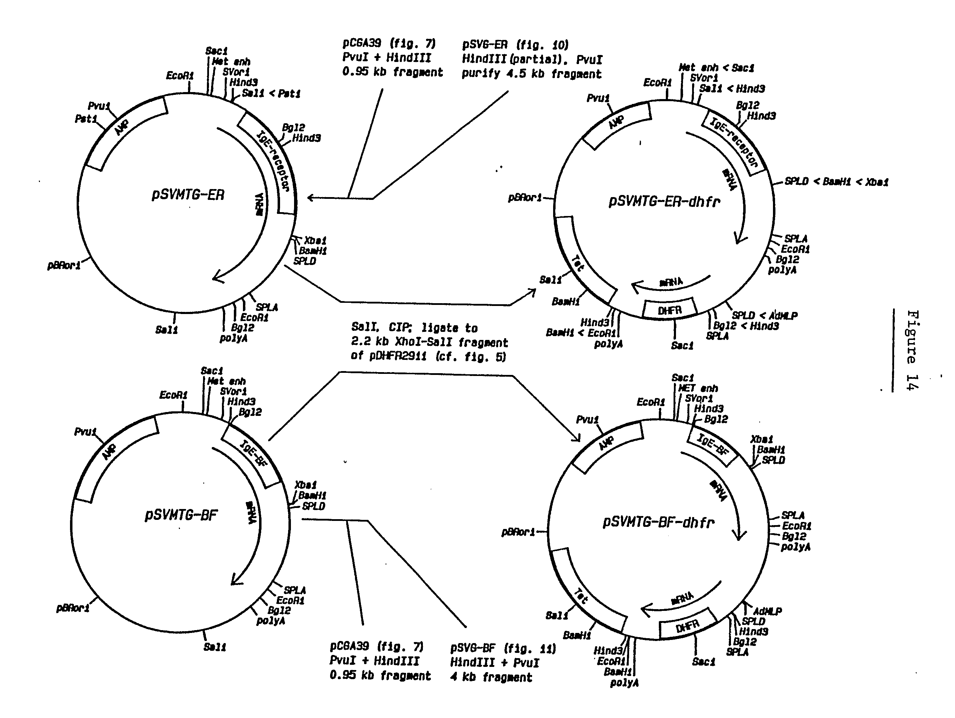

E) Insertion of DHFR gene (Fig. 5)

Plasmid pDHFR2911 [5.3 kb; F. Asselbergs et al., J. Mol. Biol. 189, 401-411 (1986)] contains a modular dihydrofolate reductase (DHFR) gene, a useful selection gene in DHFR minus cells which can also be used to co-amplify DNA sequences with the DHFR gene when transformed tissue culture cells are exposed to gradually increasing concentrations of the drug methotrexate. The 2.2 kb DHFR gene is excised from pDHFR2911 using restriction enzymes XhoI and SalI, purified via an agarose gel, and ligated to SalI cut pCGA28 (see Fig. 5). As the insert completes the truncated tetracyclin resistance gene in pCGA28, the new plasmid, designated pCGA48 (11.9 kb), is readily identified as it renders E.coli resistant to three antibiotics: ampicillin, tetracyclin and kanamycin. Plasmid pCGA48 can be used to transform DHFR-minus cells using the DHFR gene as selection marker and DHFR positive cells using the neo selection marker. Exposing such pCGA48-transformed cells to gradually increasing concentrations of methotrexate frequently leads to amplification of the chromosomally integrated pCGA48 DNA, regardless whether the originally transformed cell line contained a functional DHFR gene or not. The increased copy number of the plasmid DNA frequently results in increased protein production levels.

F) Construction of plasmid pCGA31 (Fig. 6)

Plasmid pCGA31 (6.7 kb) is constructed by cutting 10 μg pCGA28 (cf. 1D) to completion with HindIII, purifying the larger (6.7 kb) fragment and recircularizing this DNA with T4 ligase. Upon transformation pCGA31 is obtained, which contains the tPA-beta-globin unit under transcriptional control of the SV40 early enhancer/ promoter. (Fig.6).

G) Construction of plasmid pCGA31-DHFR

This plasmid is constructed (fig.6) by cutting 10 μg pCGA31 to completion with SalI followed by extraction with phenol and chloroform, after which the DNA is precipitated with alcohol and redissolved in 50 μl TE (10 mM Tris/HCl pH 8.0 containing 1 mM EDTA). 10 ng of this DNA is ligated to 10 ng of the DHFR-containing XhoI-SalI fragment of pDHFR2911 (cf. 1E), transfected into E. coli HB101 and tetracyclin-resistant colonies are selected. A colony is chosen and its restriction enzyme digestion pattern conforms to that predicted for pCGA31-DHFR (9 kb). pCGA31-DHFR contains the same gene arrrangement as pCGA48 except that the t-PA gene is driven by the SV40 early promoter and that the entire neo-unit is missing.

H) Construction of plasmid pCGA46

This section describes construction of a plasmid capable of producing t-PA under control of a hybrid enhancer/promoter consisting of an SV40 early promoter containing enhancer DNA from the mouse metallothionein I gene. pSVMT17 is a plasmid described by E. Serfling et al. [EMBO-J. 4, 3851-3859 (1985)] containing cloned into the pBR322 BamHI site a complete recombinant SV40 virus SVMT-I-17 cut at its single BamHI site. 10 μg pBRd [F. Asselbergs et al.,

J.Mol.Biol. <u>189</u>, 401-411 (1986)] a pBR322 derivative lacking the so-called poison sequence, was cut at the ClaI site and the BamHI site and the large fragment purified by gel electrophoresis. Similarly pSVMT17 was cut with HpaII and BamHI and the 3 kb fragment containing the early gene was purified. The HpaII restriction enzyme leaves the two-base overhang as the ClaI enzyme. Thus, 10 ng of the HpaII-BamHI pSVMT17 fragment was ligated to 10 ng ClaI-BamHI pBRd fragment, which upon transformation into E. coli produces plasmid pCGA39 (5.9 kb; fig.7). 10 µg of this plasmid was cut to completion with both HindIII and SalI and the large 3.4 kb fragment was purified. Similarly, plasmid pDO10 (cf. 1D) is cut to completion with HindIII, PvuI and SalI and the 3.4 kb HindIII-SalI fragment is purified. It is necessary to cut the plasmid also with a third enzyme like PvuI, as SalI plus HindIII cut the plasmid into fragments of equal size. Coligation of 10 ng of each of these two fragments, followed by transfection into <u>E. coli</u> HB101 produces plasmid pCGA46, which now contains the t-PA coding sequence with upstream the metallothionein-SV40 hybrid promoter and downstream the rabbit Beta-globin fragment necessary for proper splicing and polyadenylation of the transcript (Fig. 8).

## I) Construction of plasmid pCGA46-DHFR

This plasmid is constructed (Fig. 8) by cutting 10 µg pCGA46 to completion with SalI, followed by extraction with phenol and chloroform, after which the DNA is precipitated with alcohol and redissolved in 50 µl TE (10 mM Tris/HCl pH 8.0 containing 1 mM EDTA). 10 ng of this DNA is ligated to 10 ng of the DHFR-containing XhoI-SalI fragment of pDHFR2911 (cf. 1E), transfected into <u>E. coli</u> HB101 and tetracyclin-resistant colonies are selected. A colony is chosen and its restriction enzyme digestion pattern conforms to that predicted for pCGA46-DHFR. pCGA46-DHFR (8.6 kb) contains basically the same gene arrrangement as pCGA31-DHFR except that it contains the metallothionein-SV40 hybrid promoter instead of the standard SV40 early promoter.

Example 2: Construction of plasmids for expression of IgE-receptor and secreted IgE-binding factor.

This section describes the construction of plasmids capable of expressing the immunoglobulin type E (IgE) receptor described by Luedin et al., [EMBO J. 6, 109-114 (1987)] in mammalian tissue culture cells. Plasmids with affix-ER are intended for production of the IgE-receptor, whereas plasmids with affix-BF produce IgE-binding factor. Plasmids designated -DHFR contain in addition a dihydrofolate reductase gene as selection marker. Described are the construction of pSVG-ER, pSVG-ER-DHFR, pSVG-BF, pSVG-BF-DHFR, which all use the SV40 early enhancer/promoter, pMG-ER, pMG-ER-DHFR, pMG-BF and pMG-BF-DHFR, which use the mouse cytomegalovirus major immediate early promoter and pSVMT-ER, pSVMT-ER-DHFR, pSVMT-BF and pSVMT-BF-DHFR, which all use a derivative of the SV40 enhancer/promoter in which the main part of the enhancer, the so-called 72bp repeats, is replaced by enhancer DNA from the mouse metallothionein gene.

a. Construction of pCAL3

pCAL3 (4.1 kb) is a plasmid in which the HincII/RsaI fragment (taken from pCL2, 4.5 kb) of the IgE-receptor-cDNA (Luedin et al., supra) is cloned into pGEM™-1 (Promega Biotec., Madison, Wisconsin, USA), as follows:

10 μg of pCL-2 (isolated from E.coli HB101/pCL-2 deposited at DSM on July 30, 1986 under no. DSM3807) plasmid DNA is digested with the restriction enzymes HincII and RsaI. Fragments of 1.6, 1.25, 0.72, 0.46 and 0.23 kB are obtained and separated by electrophoresis through a 1 % agarose gel in the TBE buffer. The 1.25 kB DNA fragment is recovered as described in Example 1. In parallel, 10 μg of the plasmid pGEM™-1 (2.9 kb) are linearized with 20 units HincII (Boehringer) in 50 μl of 10 mM Tris-HCl pH 7.6, 50 mM NaCl, 10 mM $MgCl_2$ and 5 mM DTT. After 2 hours at 37°C, the reaction mixture is supplemented with 50 μl 1 M Tris-HCl pH 8.5, 10 μl $H_2O$ and 20 units alkaline phosphatase from calf intestine (Boehringer) and the incubation is continued for 30 min at 37°C. The mixture is
extracted three times with phenol-chloroform and then electrophoresed through a 1 % agarose gel in TBE buffer. The plasmid DNA is
recovered from the gel by electroelution as described in Example 1.
10 ng of the 1.25 kb cDNA fragment and 10 ng of the HincII cleaved
pGEM™-1 are ligated in a volume of 10 μl for 10 hours at 15°C. The
reaction mixture contains in addition 10 mM Tris-HCl pH 7.5,
10 mM MgCl₂, 2 mM DTT, 0.5 mM ATP and 100 units T₄-ligase
(Boehringer). 5 μl of the mixture are used to transform competent
E.coli HB101 cells as described by [T. Maniatis et al., Molecular
cloning, a laboratory manual, Cold Spring Habor Laboratory, Cold
Spring Harbor N.Y. (1982), p. 250]. The bacteria are plated on agar
plates supplemented with 100 μg/ml ampicillin. About 50 ampicillin
resistant colonies are obtained. 24 colonies are grown up and the
plasmid DNA is isolated from each culture. Approximately 1 μg of
plasmid DNA from each culture is digested with HindIII and the
length from the digested DNA fragments analysed on a 1 % agarose
gel, using HindIII digested lambda DNA (Pharmacia, Sweden) as a size
marker. The digested plasmid DNA of several colonies give DNA
fragments with 0.5 kb and 3.6 kb, several others with 0.7 kb and
3.4 kb of length, corresponding to the two possible orientations of
the fragment insertions. These plasmids are named pCAL-3 and pCAL-4
respectively (Fig. 9).

b. Construction of plasmid pSVG-ER

Plasmid pSV2911neo [F. Asselbergs, et al., J. Mol. Biol. 189,
401-411 (1986)] is used to prepare the vector DNA. It is digested
with the restriction enzymes HindIII and SalI. Fragments of 3.9 and
1.8 kb are obtained. The mixture is treated with alkaline phosphatase and the 3.9 kb fragment is isolated after electrophoresis
through a 1 % agarose gel. In parallel, two DNA inserts are prepared
which code for the IgE receptor and the 3'-half of the rabbit
beta-globin gene, respectively. On the one hand, 10 μg of plasmid
pCAL3 are digested partially with HindIII and completely digested
with BamHI. The cDNA insert of 1.26 kb is isolated by agarose gel
electrophoresis and gel elution. On the other hand, 10 μg of plasmid

pUβ (cf. Example 1B, Fig. 2) are digested to completion with BamHI and SalI. The 1.2 kb DNA fragment is isolated. It contains the large intron and the poly(A) signal of the rabbit beta-globin gene. 10 ng of the vector DNA from above and 10 ng of each insert DNA are ligated. The resulting DNA is used to transfect competent E.coli HB101. A recombinant plasmid (pSVG-ER; 5.6 kb; Fig. 10) is selected which shows the expected restriction pattern as expected after the uptake of both DNA inserts into the vector DNA.

## c. Construction of plasmid pSVG-ER-DHFR

10 ng of pSVG-ER plasmid DNA are partially digested with SalI. DNA molecules which are cut only once, and thus represent full-length linears, are purified by agarose gel electrophoresis. In parallel, 10 µg of pDHFR2911 plasmid DNA (cf. Example 1E, Fig. 5) is cleaved to completion with XhoI and SalI and the 2.2 kb DHFR-containing fragment is purified as described in example 1E. Then, 10 ng of SalI-cleaved pSVG-ER and 10 ng of the 2.2 kb SalI/XhoI fragment of pDHFR2911 are mixed and ligated. The resulting DNA is used to transform competent E.coli HB101 cells. The transformed cells are spread onto a agar plate containing LB-broth and 10 µg/ml tetracyclin. A recombinant plasmid (pSVG-ER-DHFR, 7.8 kb; Fig. 10) displaying the expected DNA restriction pattern is chosen for the expression of the IgE receptor in mammalian hosts. It contains the dhfr-selection markers downstream of the IgE-receptor cDNA. The direction of transcription is the same for both genes.

## d. Construction of plasmid pSVG-BF

The plasmid pSVG-BF (5 kb) is a derivative of the plasmid pSVG-ER described above, wherein the DNA sequence coding for amino acids 1 to 147 of the polypeptide of formula I are replaced by a new DNA sequence coding for the signal sequence of the avian influenza hemagglutinin. This change allows the secretion of IgE-binding factors comprising amino acids 148 to 321 of the polypeptide of formula (single letters refer to the common abbreviations of the encoded L-amino acids)

```
1                    10                   20                   30
M  E  E  G  Q  Y  S  E  I  E  E  L  P  R  R  R  C  C  R  R  G  T  Q  I  V  L  L  G  L  V

                     40                   50                   60
T  A  A  L  W  A  G  L  L  T  L  L  L  L  W  H  W  D  T  T  Q  S  L  K  Q  L  E  E  R  A

                     70                   80                   90
A  R  N  V  S  Q  V  S  K  N  L  E  S  H  H  G  D  Q  M  A  Q  K  S  Q  S  T  Q  I  S  Q

                     100                  110                  120
E  L  E  E  L  R  A  E  Q  Q  R  L  K  S  Q  D  L  E  L  S  W  N  L  N  G  L  Q  A  D  L

                     130                  140                  150
S  S  F  K  S  Q  E  L  N  E  R  N  E  A  S  D  L  L  E  R  L  R  E  E  V  T  K  L  R  M

                     160                  170                  180
E  L  Q  V  S  S  G  F  V  C  N  T  C  P  E  K  W  I  N  F  Q  R  K  C  Y  Y  F  G  K  G

                     190                  200                  210
T  K  Q  W  V  H  A  R  Y  A  C  D  D  M  E  G  Q  L  V  S  I  H  S  P  E  E  Q  D  F  L

                     220                  230                  240
T  K  H  A  S  H  T  G  S  W  I  G  L  R  N  L  D  L  K  G  E  F  I  W  V  D  G  S  H  V

                     250                  260                  270
D  Y  S  N  W  A  P  G  E  P  T  S  R  S  Q  G  E  D  C  V  M  M  R  G  S  R  W  N  D

                     280                  290                  300
A  F  C  D  R  K  L  G  A  W  V  C  D  R  L  A  T  C  T  P  P  A  S  E  G  S  A  E  S  M

                     310                  320
G  P  D  S  R  P  D  P  D  G  R  L  P  T  P  S  A  P  L  H  S     .
```

To this end, a ds DNA fragment is chemically synthesized according to standard methods described in literature [cf. S.A. Narang et al., Anal.Biochem. 121, 365 (1982); K.L. Agarwal et al., Angew.Chem. 84, 489 (1972); C.B. Reese, Tetrahedron 34, 3143 (1972); R.L. Letsinger et al., J.Am.Chem.Soc. 98, 3655 (1976); Khorana et al., J.Biol.Chem. 251, 565 (1976); S.A. Narang, Tetrahedron 39, 3 (1983)] having the formula

```
5'-pAGCTTACCATGGCTACCCAGATCTTGGTGTTCGCTCTGGTGGCCGTCATTCCTACAAACGCGC
       ATGGTACCGATGGGTCTAGAACCACAAGCGAGACCACCGGCAGTAAGGATGTTTGCGCGATTP-5'
```

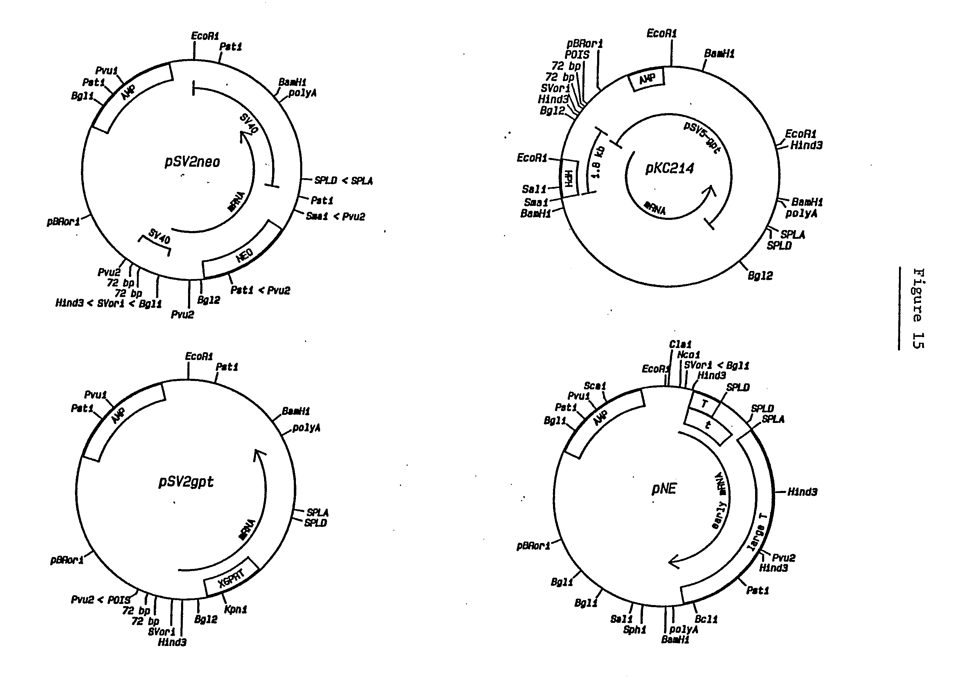

0.2 ng of this DNA is ligated with 10 ng of vector DNA and 2 ng of a 0.7 kb DdeI/XbaI cDNA insert. The vector fragment is obtained from pSVG-ER (Fig. 10) plasmid DNA by sequentially: digestion to completion with XbaI and HindIII, dephosphorylation with alkaline phosphatase, and agarose gel purification of the vector DNA. The 0.7 kb cDNA insert fragment is derived from pCAL3 (Fig. 9). 30 µg of pCAL3 plasmid DNA are digested with HindIII and XbaI, and the 0.8 kb cDNA insert is isolated by agarose gel purification. 3 µg of this 0.8 kb fragment are digested with DdeI, thereby producing DNA fragments of 0.1 and 0.7 kb. Finally the 0.7 kb DNA fragment is isolated by agarose gel electorphoresis and purification.
The ligated DNA is used to transform competent E.coli HB101 cells. A recombinant plasmid (pSVG-BF; Fig. 11) is selected which shows the expected restriction fragments, as expected after the integration of the DNA insert into the vector DNA.


e. Construction of pSVG-BF-DHFR

10 µg of pSVG-BF plasmid DNA are digested to completion with SalI. The linear DNA molecules are purified by agarose gel electro- phoresis. 10 ng of SalI cleaved pSVG-BF are ligated with 10 ng 2.2 kb SalI/XhoI-fragment of plasmid pDHFR2911. The ligated DNA is used to transform competent E.coli HB101 cells which are plated on a agar plate containing LB-medium supplemented with 50 µg/ml tetracyclin. A recombinant plasmid (pSVG-BF-DHFR; 7.3 kb; Fig. 11) is selected for the expression and production of IgE-BFs in transformed mammalian hosts.


f. Construction of plasmid pMG-ER


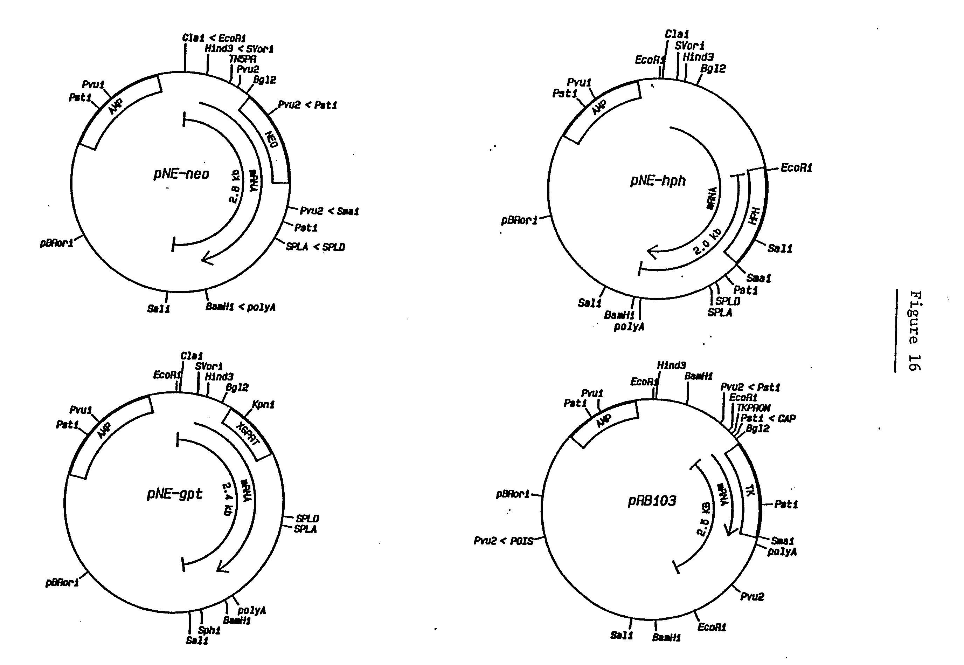

10 µg pSP62Pst33 (Fig. 12), a plasmid containing a 2.1 kb PstI fragment of the mouse cytogalovirus (MCMV) DNA, which includes the viral major immediate-early (IE) promoter, inserted into the PstI site of plasmid pSP62 (Boehringer-Mannheim) is cut with AccI. The base overhang resulting after cutting with the restriction enzyme is filled in with E. coli (large fragment) DNA polymerase I, the DNA polymerase is then removed by extracting the DNA with phenol, with chloroform, followed by alcohol precipitation of the DNA. Subsequently the DNA is redissolved, digested to completion with HindIII restriction enzyme after which the 0.5 kb fragment containing the MCMV IE enhancer/promoter is purified by gel electrophoresis. Similarly, 10 µg of plasmid pSVG-ER (Fig. 10) are cut with EcoRI, the base overhangs are filled in with DNA polymerase I, the DNA is then extracted with phenol and chloroform, precipitated with alcohol, after which the DNA is digested with SalI and the largest fragment, containing the beta-globin splice/polyadenylation unit, the bacterial replication orgin and the ampicillin resistance gene is isolated. A ligation reaction is now set up, in which 10 ng of this blunt(ex-EcoRI)-BamHI fragment is ligated with 10 ng of the blunt(ex-AccI)-HindIII MCMV enhancer/promoter fragment and 10 ng of the HindIII(partial)-BamHI fragment from pCAL3 (cf. example 2b). The ligated DNA is transfected into E. coli HB101 and colonies are isolated. By restriction enzyme analysis a colony is identified in which the blunt ends of the filled AccI and EcoRI sites are ligated to each other and in which the HindIII sites of the two parent plasmids have become connected. Thus, in the resultant plasmid pMG-ER (5.7 kb; Fig. 12) the relatively weak SV40 early enhancer/ promoter in pSVG-ER is now replaced by the MCMV enhancer/promoter, which in many cells induces a higher level of transcripts.

g. Construction of plasmid pMG-ER-DHFR

10 µg of plasmid pMG-ER are partially digested with SalI and linear DNA is purified by gel electrophoresis. To 10 ng of this DNA is ligated 10 ng of the 2.2 kb XhoI-SalI fragment of pDHFR2911 containing the DHFR selection marker. This ligated DNA is transfected into E. coli HB101 and tetracyclin resistant colonies are obtained as in Example 2e. The resulting plasmid pMG-ER-DHFR (7.9 kb; Fig. 12) can be transfected into mammalian tissue culture cells to obtain cell lines that secrete IgE-binding factor. The copy number of the plasmid in the tissue culture cells can be increased from that in the original transformant by selection for resistance to increasing concentration of the antifolates such as methotrexate frequently with a proportial increase of the level IgE-BF produced.

## h. Construction of plasmid pMG-BF

10 µg pMG-ER are cut to completion with HindIII and XbaI and the largest fragment (4.3 kb) comprising the the MCMV enhancer/promoter, the beta-globin splicing/polyadenylation fragment, the ampicillin gene plus the bacterial origin of replication is purified by gel electrophoresis. To 10 ng of this DNA is ligated 10 ng of the small HindIII-XbaI fragment of pSVG-BF containing the coding DNA for a secretory IgE-binding factor (cf. Example 2d). After transfection into E. coli HB101 an ampicillin resistant colony is isolated, DNA from which has the predicted restriction enzyme pattern and is designated pMG-BF (5.2 kb; Fig. 13).

## i. Construction of plasmid pMG-BF-DHFR

10 µg of plasmid pMG-BF are digested with SalI and linear DNA is purified by gel electrophoresis. To 10 ng of this DNA is ligated 10 ng of the 2.2 kb XhoI-SalI fragment of containing the DHFR selection marker. This ligated DNA is transfected into E. coli HB101 and tetracyclin resistant colonies are obtained as in Example 2e. The resulting plasmid pMG-BF-DHFR (7.4 kb; Fig. 13) can be transfected into mammalian tissue culture cells to obtain cell lines that secrete IgE-binding factor. The copy number of the plasmid in the tissue culture cells can be increased from that in the original transformant by selection for resistance to increasing concentration of the antifolates such as methotrexate frequently with a proportional increase of the level IgE-BF produced.

## j. Construction of plasmid pSVMTG-ER and pSVMTG-ER-DHFR

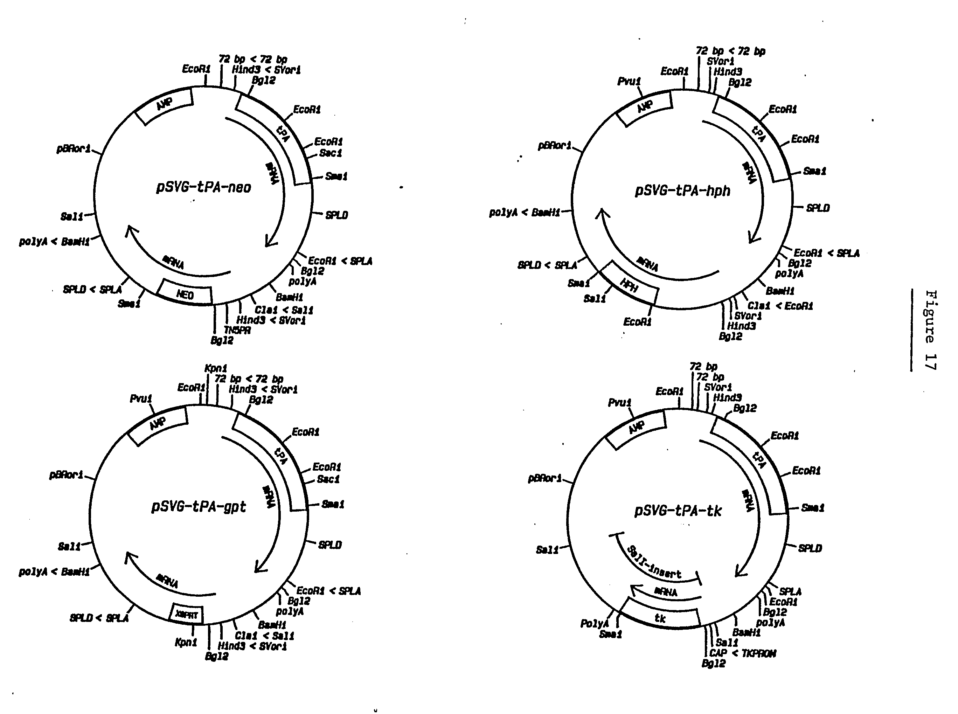

pCGA39 is a plasmid containing an SV40 early gene in which its 72 bp repeats are replaced by enhancer DNA from the mouse metallothionein-I gene (cf. Example 1H). It is constructed by cloning the HpaII-BamHI fragment containing the early gene unit from recombinant SV40 virus SVMT-I-17 (Serfling et al., supra). 10 µg pCGA39 are cut to completion with PvuI and HindIII and the 0.95 kb fragment containing the hybrid enhancer/promoter is purified. In addition from 10 µg pSVG-ER (Fig. 10) cut partially with HindIII the linear form

- 32 -

of the plasmid is purified, which is then cut to completion with PvuI. Thus, a PvuI-HindIII fragment of 5 kb is obtained containing all of pSVG-ER except the enhancer/promoter. 10 ng of this fragment is now ligated to 10 ng of the 0.95 kb fragment of pCGA39 (Fig. 7), which upon transfection into E. coli HB101 yields plasmid pSVMTG-ER (5.5 kb; Fig. 14). In analogy to the procedure described above for the construction of pSVG-ER-DHFR, tetracyclin resistance plasmid pSVMTG-ER-DHFR (7.7 kb) is constructed by inserting the XhoI-SalI DHFR fragment from pDHFR2911 into pSVMTG-ER linearized by a partial cut with SalI (Fig. 14).

### k. Construction of plasmids pSVMTG-BF and pSVMTG-BF-DHFR

As described above from 10 µg pCGA39 (Fig. 7) cut to completion with PvuI and HindIII is purified the 0.95 kb fragment containing the hybrid enhancer/promoter. In addition 10 µg pSVG-BF is cut to completion with HindIII and PvuI and the large fragment containing all of pSVG-ER except the enhancer/promoter is purified (Fig. 11). 10 ng of this fragment is now ligated to 10 ng of the 0.95 kb fragment of pCGA39, which upon transfection into E. coli HB101 yields plasmid pSVMTG-BF (5 kb; Fig. 14). In analogy to the procedure described above for the construction of pSVMTG-ER-DHFR, tetracyclin resistance plasmid pSVMTG-BF-DHFR (7.2 kb) is constructed by inserting the XhoI-SalI DHFR fragment from pDHFR2911 into pSVMTG-BF linearized by cutting to completion with SalI (Fig. 14).

### Example 3: Production of t-PA by transformed mammalian cells

### A) Maintenance and DNA transfection of tissue culture cells; general procedure

The methods used for transfection of DNA into mammalian cells, as well as selection of transformed cells and selection for gene amplification have been described in detail [US Patent 4,399,216; R.J. Kaufman and P.A. Sharp, J.Mol.Biol. 159, 601-621 (1982)] and are well known to one skilled in the art.

DNA constructs are expressed in Chinese hamster ovary (CHO) cells of line DUKXB1, a mutant lacking the enzyme dihydrofolate reductase [G. Urlaub et al., Proc. Nat. Acad. Sci. USA 77, 4216-4220 (1980)]. The cells are cultured in alpha-MEM medium containing nucleosides (GIBCO) supplemented with 5 % fetal calf serum.

Cells are plated at a density of 10 000/cm$^2$ in 6-well multiplates (3.4 cm diameter) and transformed with 4 µg DNA: DNA is dissolved at 50 µg/ml in 10 mM Tris/HCl pH 7.0 containing 0.1 mM EDTA, cooled on ice for 5 min., 0.25 volumes 1 M CaCl$_2$ is added and incubated on ice for 10 min. The mixture is then mixed with an equal volume of 2 x HBS (50 mM Hepes, 280 mM NaCl, 0.75 mM Na$_2$HPO$_4$, 0.75 mM NaH$_2$PO$_4$. pH 7.12) followed by another 10 min incubation on ice. Finally this DNA-Ca-phosphate coprecipitate is added to the culture medium and cells are incubated with the DNA for 16-18 h, followed by a glycerol shock, i.e. cells are rinsed with TBS (80 g/l NaCl, 3.8 g/l KCl, 1 g/l Na$_2$HPO$_4$.2H$_2$O, 0.114 g/l CaCl$_2$.2H$_2$O, 0.11 g/l MgCl$_2$.6H$_2$O, 25 mM Tris/HCl pH 7.5), incubated 1 min with 20 % (v/v) glycerol in TBS, rinsed again with TBS and cultured 24 h in tissue culture medium. Cells are then trypsinized and the cells are transferred to three 8 cm diameter Petri dishes. The next day the initial non-selective culture medium is replaced by medium without nucleosides and with 5 % dialyzed FCS instead of normal FCS. Medium is replaced every third or fourth day. Colonies can be seen around day 14. Cells from individual colonies are isolated by scraping them off with the tip of a pipetman while simultaneously sucking them into the tip filled with trypsin solution and transferring each to a well of a 24 well multiplate supplied with selective medium. When these are confluent, the culture medium is replaced by medium without serum and 24 h later aliquot of the conditioned medium is taken in order to test its content of human t-PA.

## B) Agarose plate-assays for plasminogen activator

A sensitive assay for plasminogen activator uses agarose gels to
which plasminogen (stock solution prepared by dissolving plasminogen
Sigma A-6877 at 1 mg/ml in and dialyzing it twice against
100 volumes 50 mM Tris/HCl pH 8.0) and either casein (added as
non-fat milk) or fibrin (added as fibrinogen plus thrombin) is
added. The sample containing plasminogen activator, such as 0.025 ml
of the conditioned medium described above, is applied into 5 mm
diameter holes punched into a 4 mm thick agarose layer and the gel
is subsequently incubated at 37°C. The enzymatic activity is then
detected in that the plasminogen activator diffuses radially away
from the sample well, converts the plasminogen in the gel to plasmin
which in turn digests the casein or fibrin thus producing a clear
halo in the opaque gel around the sample well. The radius of the
halo (most conveniently measured from the rim of the sample well) is
a measure for the amount of plasminogen activated. The assay does
not shown a linear response to the amount of plasminogen activator
added. For assay of low amounts plasminogen activator the incubation
can be prolonged to several days. The procedure and calibration of
the casein assay is as described in J. Tang et al.,
[Ann.N.Y.Acad.Sci 434, 536-540 (1984)] except that instead of
2 % (w/v) Carnation non-fat milkpowder 12.5 % (v/v) sterilized (UHT)
fat-free milk from Migros Corp. (Switzerland) is used. When fibrin
[A. Granelli-Piperno and E. Reich, J.Exp.Med. 148, 223-234 (1978)]
is used as a substrate 0.2 g agarose is dissolved in 15 ml 0.9 %
NaCl and cooled to 42°C. At this point 5 ml 0.9 % NaCl containing
80 mg bovine fibrinogen (Sigma F-8630), 0.1 ml plasminogen solution
(above) and 0.1 ml 100 mg/ml NaN$_3$ at 42°C are added. Finally, 0.2 ml
bovine thrombin (Sigma T-6634, dissolved at 16.6 NIH units/ml in
0.9 % NaCl) are added and the mixture is quickly poored into a Petri
dish (8 cm diameter) and allowed to cool to room temperature for an
hour. The resulting resulting gel is about 4 mm thick and like the
casein containing gel can be stored at 4°C for several days or used
immediately in the same manner as described above.

C) <u>Fluorometric quantitation of PA activity</u>

Conditioned medium from a known number of cells is isolated as
described in A). The amidolytic activity is then measured according
to M. Zimmerman et al. [Proc.Natl.Acad.Sci. USA <u>75</u>, 750-753 (1978)]
using N-CBZ-glycyl-glycyl-L-arginine-7-amino-4-methyl-coumarin
(Bachem, Dübendorf, Switzerland) as fluorogenic substrate and
7-amino-4-methyl-coumarin (Sigma) as standard.

D) <u>Efficiency of transformation to t-PA production</u>

Following the procedure described in Example 3A more than
50 DHFR$^+$ colonies are obtained per plate. Well-developed colonies
are randomly chosen and amplified in 24 well microtiter culture
plates (NUNC). At confluency standard medium is replaced by medium
(Alpha-MEM without nucleosides, GIBCO) without serum and 24 h later
conditioned medium is harvested and tested for its content of human
tPA. Of 48 colonies thus investigated all produced a halo of at
least 4 mm on a casein plate after an 18 h incubation. Under these
circumstances no halo is observed with conditioned medium from
untransformed CHO DUKXB1 cells or control cells transformed by
plasmid pAdD26SVpA (Kaufman and Sharp, supra), which does not encode
a plasminogen activator. Thus it is concluded that more than 95 % of
the DHFR$^+$ CHO cells transformed by pCGA48 also produce human t-PA.
This result is confirmed on fibrin agarose plates, which generally
take only 4-6 h to develop. On such plates after prolonged incubation (18 h) a tiny halo of less than 1 mm is observed due to the
presence of a minimal amount of hamster t-PA produced by the
CHO cells. That the amount of human t-PA far exceeds the production
of hamster t-PA is also evidenced when conditioned media are tested
fluorometrically. The average production observed is
22 ± 10 pg/cell/24h relative to a laboratory standard t-PA isolated
from Bowes melanoma cells with a specific activity of 45,000 FU/mg
protein (FU: fluorometric units). Furthermore, in this test which is
described in Example 3C conditioned medium from pCGA48-transformed DHFR$^+$ CHO cells scores at least 10 times higher than that of control CHO cells. DHFR$^+$ CHO cells still produce human t-PA after being subcultured for 6 weeks i.e. after about 50 cell generations indicating that t-PA production is due to stable integration into the host chromosome.

E) Calculation of the expected efficiency of t-PA production

In the above example, pCGA48 DNA is applied as circular plasmid DNA. As part of the intracellular process of integration of the plasmid into the host chromosome, the plasmid in converted to a linear form. As this is thought to occur by a cut at a random site in the DNA, the chance that the DNA sequence of a gene is interrupted as result of the linearization is a primarily a function of the size of the gene. In the case of selection of DHFR$^+$ colonies, only linearization events outside the 2 kb DHFR gene produce viable progeny. If the 9.6 kb DNA of pCGA48 outside the DHFR gene is considered 100 %, than the 3.9 kb t-PA unit, the 2.8 kb neo unit and the 2.9 kb of pBR322-derived DNA constitute 41 %, 29 % and 30 % respectively. Thus, only 59 % of the linearization events outside the DHFR gene are expected to occur in the neo unit or in the pBR322-derived DNA leading to a t-PA producing colony, whereas in 41 % of the cases the t-PA would probably be damaged. Out of 48 colonies chosen, therefore, more than 10 would be expected not to produce tPA.

F) Explanation of the unexpectedly high frequency of t-PA
   production

As shown in Example 3D, the expected 41 % non-t-PA-producing colonies are not observed. Note, that as a result of a hypothetical linearization event inside the t-PA gene, the spatial linkage between the enhancer, present in the MCMV enhancer/promoter unit of the t-PA gene and the adenovirus promoter, which is also dependent on stimulation by the MCMV enhancer is disrupted. Therefore, such linearization events as a rule do also produce non-viable DHFR-transformants. Thus, due to to the special spatial arrangement of the genetic elements in pCGA48 this plasmid produces upon transformation a population of CHO cells of which an extremely high
proportion if not all produce t-PA.

G) Transformation of CHO cells with pCGA31-DHFR

Transfection of DHFR-CHO cells occurs as descibed in Example 3A.
Slightly fewer colonies are obtained than with pCGA48. 48 colonies
are chosen for evaluation of t-PA production as described in
Examples 3B and 3C. Production of t-PA is observed in 47 colonies.
At 15 ± 3 pg/cell/24h the average production is slightly lower than
observed with pCGA48.

H) Transformation of CHO cells with pCGA46-DHFR

Transfection of pCG46-DHFR under the standard conditions described
in Example 3A yields few or no colonies at all because without
appropriate stimulation of the metallothionein enhancer the DHFR
unit is sufficiently active to allow survival of cells under
selective conditions. Selective conditions are, therefore, slightly
changed in that $5 \times 10^{-5}$ M ZnSO4 and $5 \times 10^{-7}$ M CdSO4 are added
(optimal under our conditions). If ZnSO4 is omitted cells are killed
by the CdSO4 presumably due to insufficient production of metallothionein. If too much CdSO4 is added cells also die. Both the toxic
concentration and the optimal concentration for cloning vary with
the exact medium conditions and also depend on the serum batch used.
Concentrations higher than $2 \times 10^{-7}$ M CdSO4 should usually not
being used. At least 50 clones/µg plasmid DNA are obtained. Of
48 picked colonies at least 95 % express t-PA in the presence of
optimal amount of metal salts. In more than 25 % of the clones t-PA
and DHFR synthesis are controlled in a metal-dependent manner. When
the CdSO4 concentration is lowered t-PA synthesis by metal-
responsive cells decreases and in medium selecting for DHFR expression growth of the cells slows down. Conversely, the rate of
t-PA synthesis increases at higher CdSO4 concentrations reaching
levels equal or higher than the constitutive production level of pCGA31-DHFR transformed cell lines. The metal-stimulated t-PA synthesis is at least 5 times higher than in the absence of CdSO4. The regulated synthesis of t-PA and DHFR is maintained when by selection in gradually increasing concentrations of methotrexate the plasmid copy number is increased. The advantage is that later in the amplification process at lower CdSO4 concentrations also less methotrexate is needed to maintain selective pressure. Thus by performing further amplification at low t-PA production levels due to low CdSO4 levels, cell lines can be obtained which when stimulated by CdSO4 are capable of producing amounts of t-PA which would have preclude actual growth of the cells.

## Example 4: Selection of transformed mammalian cell lines expressing polypeptides with IgE-receptor or -binding activity

### A) CHO Cells producing IgE-bindung factor (IgE-BF)

A dihydrofolate reductase negative mutant (dhfr$^-$) of a Chinese hamster ovary cell line is used as host [Cell line DUKX-B1; L.A. Chasin and G. Urlaub, Proc. Nat. Acad. Sci. USA 77, 4216-4220 (1980)]. The cells are maintained in MEM-alpha medium (Gibco, Paislay, Scotland) supplemented with 5 % FCS and 0.1 mg/ml gentamycin (Gibco). Subconfluent CHO cells are transfected with 4 µg pSVG-BF-DHFR or pMG-BF-DHFR plasmid DNA using the Ca-phosphate co-precipitation method as described in Example 3A. The transfected cells are grown in Alpha-MEM medium without nucleosides supplemented with 5 % dialyzed and FCS gentamycin.

After two weeks, single DHFR$^+$ colonies grow out. 48 colonies are transferred individually into the wells of a 24-well microtiter plate (NUNC) and grown to confluency in the selection medium described above. Then, aliquots of the medium are removed and tested for the presence of IgE-binding factor using the RIA described below. In 3 independent transformation experiments, each time more than 95 % of the colonies are positive and produce more than 1-10 ng of recombinant polypeptide per ml. Plasmid pMG-BF-DHFR produces significantly more colonies than pSVG-BF-DHFR which produces about 100 colonies/µg DNA. pMG-BF-DHFR-transformants generally produce more than three times as much IgE-BF (1-3 ng/ml) as cells transformed by pSVG-BF-DHFR. The best producer, cell line CHO-BF, is grown into bulk cultures. Approximately $3 \times 10^6$ cells are seeded per tissue culture flask (Falcon, 175 cm$^2$) with 50 ml of selection medium. After the cell layer has reached confluency, the medium is removed and replaced with 50 ml of Alpha-MEM medium supplemented with 1 % FCS and 0.1 mg/ml gentamycin. The medium is collected twice per week for several weeks. It is centrifuged for 10 min at 5000 g and stored at -20°C. Finally, recombinant polypeptides with IgE-binding activity are purified from the combined supernatants by affinity chromatography.

B) Enzyme-linked-immuno-sorbent-assay (ELISA) of IgE-BF
Samples are assayed for IgE-BF in the following way:
PVC microtiter plate wells are coated with Mab-176 (EP 205 405; 5 µg/ml in PBS; 100 µg per well) overnight in a humidity chamber at room temperature. The plates are washed twice with PBS before nonspecific binding sites are blocked with PBS containing 0.2 % gelatine (150 µl/well; 1 h at 37°C). Plates are washed again twice with PBS. Fractions from chromatography experiments are diluted 1:50 in PBS, added to the wells (100 µl/well) and incubated overnight at room temperature in a humidity chamber. Plates are washed 4 times with PBS. Bound IgE-BF is detected by adding Mab-135 to which biotin had been attached covalently (Example 19, EP 205 405) (0.5 µg/ml in PBS containing 0.2 % gelatine; 100 µl/well) and incubated 4 h at 37°C in a humidity chamber. After washing with PBS (4 times) the plates are incubated with 100 µl of a conjugate of avidin and alkaline phosphatase (Sigma Cat. No. A 2527 0.5 µg/ml) in PBS containing 0.2 % gelatine for 2 h at 37°C. After additional washing with PBS the plates are developed with 1 mg/ml p-nitrophenyl phosphate disodium in substrate buffer (100 mg $MgCl_2 \times 6\ H_2O$, 200 mg $NaN_3$, 97 ml diethanolamin dissolved in 800 ml $H_2O$, pH adjusted to 9.8 with 37 % HCl). The yellow colour reaction is optimal after 20-40 min at 37°C. The reaction is then stopped with 50 µl NaOH
(1 M) per well. The optical density is determined using a
Model 2550 EIA Reader (Bio-Rad) at 405 mm.

C) Purification of natural IgE-BF from transformed CHO cells

Purification of IgE-BF follows essentially the protocol developed
for IgE-BF from RPMI 8866 cells supernatants (EP 205 405).

10 liter culture supernatant from CHO-BF cells are filtered through
a 20 ml column of Mab-45-affigel (Example 10 of EP 205 405) at a
flow rate 120 ml/h. The gel is washed with PBS. The protein content
of the flow-through is monitored by on-line absorbance at 280 nm by
means of a Uvicord$^R$ spectrophotometer to ensure complete removal of
unbound proteins. The column is eluted with 50 ml 0.1 M glycine-HCl,
pH 2.6. Fractions are collected in tubes containing an equal amount
of 1 M Tris-HCl, pH 8.0 and 0.5 % Tween 20. IgE-BF containing
fractions are pooled and dialyzed against 10 mM Tris-HCl, pH 7.4.

D) Purification of IgE-BF by ion exchange chromatography

Purified IgE-BF of Example 4C is loaded on a SynChropak AX 300 anion
exchanger column (SynChrom Inc., Linden, IN). The column is washed
with 10 mM Tris-HCl, pH 7.4, and the protein is eluted with a
gradient of 0 to 1 M NaCl in 100 min at a flow-rate of 1 ml/min.
Elution is monitored for UV absorbance at 254 nm. The fractions are
collected into 1 % octylpyranoglucoside (Sigma) and assayed for
IgE-BF as described in Example 4B.

E) Further purification of IgE-BF by reverse phase chromatography

50 ml of IgE-BF of Example 4D is dialyzed against 0.5 l PBS and
twice against 0.5 l PBS containing 0.1 % octylpyranoglucoside. After
lyophylization the IgE-BF is solubilized in 1.5 ml $H_2O$ and again
dialyzed against 2 x 0.5 l PBS containing 0.1 % octylpyranogluco-
side. Reversed phase chromatography is performed on a Syn-
Chropak RP-4 (SynChrom) column in 0.1 % TFA (Pierce) and 5 % acetonitrile (Merck). Elution of IgE-BF is obtained by applying a gradient of 5-60 % acetonitrile in 0.1 % TFA during 30 min at a flow-rate of 0.5 ml/min. Elution is monitored for UV absorbance at 254 nm. 1 ml fractions are collected into 0.05 % SDS and assayed for IgE-BF as described in Example A. The purity of IgE-BF is controlled by SDS-PAGE with subsequent silver staining (EP 205 405, Example 22).

Analogously plasmids pMG-ER-DHFR and pSVG-ER-DHFR are transfected into DHFR- CHO cells. Note that the frequency of production of the IgE-receptor can be assayed in two ways. First, one can use the RIA assay described hereafter to measure the presence in the conditioned medium of IgE-BF which is produced by proteolytic cleavage of IgE-receptor. Alternatively, IgE-coated bovine erythrocytes can be used produced rosettes around IgE-receptor producing CHO cells (Luedin et al., supra). By both assays more than 95 % of the $DHFR^+$ CHO clones produce IgE-receptor. The level of IgE-BF produced by these clones is much lower than by the plasmid descibed above, generally 0.1-3 ng/ml.

## F) Transfection with metal-responsive plasmids

Transfection with plasmids pSVMTG-ER-DHFR and pSVMTG-BF-DHFR is performed analogously to Example 4A exept that $5 \times 10^{-5}$ M ZnSO4 and $5 \times 10^{-7}$ M CdSO4 are added to the selective medium. Without CdSO4 only few or no colonies at all are obtained. With optimal amounts of CdSO4 at least 50 colonies are obtained per microgram DNA transfected. At least 95 % of the colonies express the IgE-receptor or IgE-binding factor respectively. The production in the presence of CdSO4 is generally equivalent or better than by cells transformed by the corresonding plasmids with the wild-type SV40 enhancer promoter. In at least 25 % of these colonies the synthesis of the IgE-receptor or IgE-binding factor is regulatable by changing the CdSO4 concentration in the manner described for the tPA-encoding plasmids in Example 3H.

Example 5: Plasmids containing other selection genes

Basis for these plasmids is a construct containing an SV40 early gene without enhancer DNA (pNE). Subsequently is described how the T-antigen coding sequences can be replaced by coding sequences for neomycin phosphotransferase (pNE-neo), xanthine-guanine-phosphoribosyltransferase (XGPRT) (pNE-gpt) and thymidine kinase (pNE-tk) and how these selection genes can be combined with the t-PA production gene.

A) Construction of plasmid pNE, pNE-neo, pNE-gpt and pNE-hph

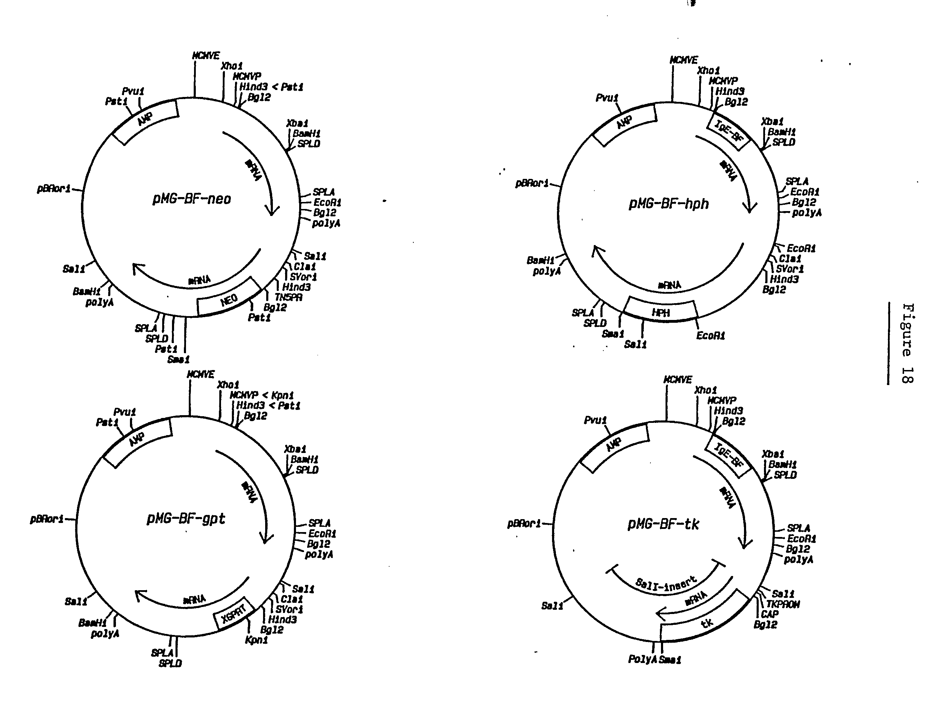

An enhancerless SV40 early gene is cloned as follows. 10 µg SV40 viral DNA is cut using SphI, the resulting staggered DNA termini are blunted using S1 nuclease, after which the DNA is purified by sequential extractions with phenol and chloroform and alcohol precipitation. Phosphorylated ClaI linkers (CATCGATG; New England Biolabs, Inc.) are ligated to the DNA which is then cut with ClaI and BamHI. Of the resulting fragments the largest is purified by gel electrophoresis and ligated to the similarly purified large ClaI-BamHI fragment of pBRd (Example 1H, Fig. 7) resulting in formation of plasmid pNE (5.8 kb; no enhancer, Fig. 15). In order to prepare this plasmid for insertion of selection genes, 10 µg DNA of this plasmid is cut to completion with HindIII and BamHI and the large fragment containing the enhancer-less promoter plus the pBR322-derived sequences is purified. Similarly, pSV2neo [5.7 kb; ATCC 37149; P.J. Southern and P. Berg, J.Mol.Appl.Genet. 1, 327-341 (1982)] is cut to completion with HindIII and BamHI and the 2 kb fragment containing sequentially the Tn5 promoter, the neomycin phosphotransferase coding sequence and a splicing/polyadenylation unit derived from SV40 t-antigen gene is purified. 10 ng of each of these fragment is coligated to yield upon transfection into E. coli pNE-neo (5.5 kb). pNE-gpt (5.0 kb) is constructed in analogous fashion except that the small HindIII-BamHI fragment of pSV2gpt [5.2 kb; ATCC 37145; R.C. Mulligan and P. Berg, Mol.Cell.Biol. 1, 449-459 (1981)] is ligated to the pNE HindIII-BamHI fragment. To form pNE-hph (6.3 kb), first pNE-neo is cut with SmaI and HindIII and the larger DNA fragment is purified. In parallel a HindIII-SmaI fragment is prepared from pKC214 [16.6 kb; Fig. 15; R.F. Santerre et al. Gene 30, 147-156 (1984)] and ligated to the pNE-neo DNA prepared above. The resultant plasmid pNE-hph is shown in Fig. 16.

B) Combination of selection genes with production gene

10 µg of plasmid pCGA31 or pMG-BF are cut to completion with SalI, cut DNA is dephosphorylated using calf intestinal alkaline phosphatase (CIP) and linear DNA is purified by agarose gel electrophoresis. This will further be called the SalI vector DNA. In parallel fragments with SalI ends containing the selection genes are prepared as follows. 10 µg linear DNA are prepared of pNE-neo, pNE-gpt and pNE-hph by cutting with EcoRI (of pNE-hph, which contains 2 EcoRI sites a partially cut DNA is prepared). Subsequently the protruding EcoRI end is converted to a blunt end by filling in using large fragment E. coli DNA polymerase I.

pRB103 [7.8 kb; L. Post et al., Proc.Natl.Acad.Sci. U.S.A. 77, 4201-4205, (1980)] contains the HSV thymidine kinase (tk) gene in the form of the HSV-1(F) BamHI-Q fragment cloned into the BamHI site of pBR322 in such a way that the direction of transcription of the tk gene is the same as that of the pBR322 Tet gene (Fig. 16). A suitable 2.5 kb thymidine kinase fragment is prepared from the plasmid after cutting with EcoRI and addition of SalI linkers. To the blunt ends thus produced SalI linkers (GGTCGACC) were ligated upon which the DNA was extracted sequentially with phenol and chloroform, precipitated with ethanol and cut to completion with SalI. Thus SalI fragments of 3 kb, 3 kb, 9 kb and 2.5 kb of pNE-neo, pNE-gpt and pRB103 (also cut with EcoRI to enable proper separation from the unwanted DNA fragment) containing each of the selection genes were obtained, further purified by gel electrophoresis, ligated to the dephosphorylated SalI vector DNA which after transfection into E. coli produces ampicillin resistant clones, respec-

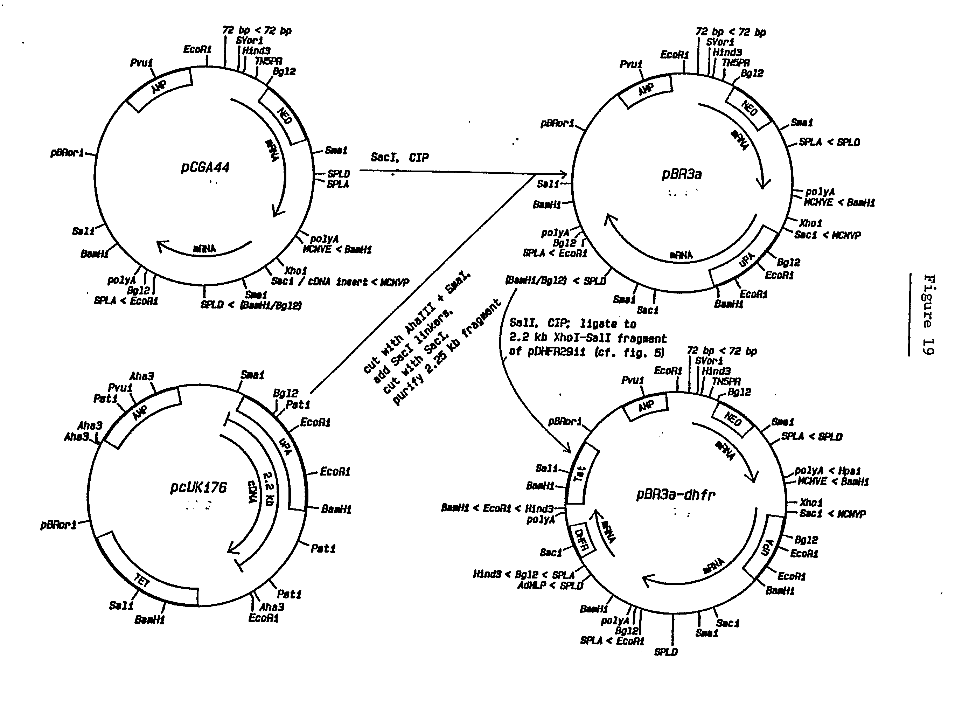

tively identified as pSVG-tPA-neo (9.6 kb), pSVG-tPA-gpt (9.2 kb), pSVG-tPA-hph (10 kb) and pSVG-tPA-tk (9.2. kb; Fig. 17), and pMG-BF-neo (8.0 kb), pMG-BF-gpt (7.6 kb), pMG-BF-hph (7.6 kb) and pMG-BF-tk (7.6 kb), respectively (Fig. 18).

The hygromycin-B resistance gene from pNE-hph is prepared in a slightly different way. First pNE-hph is linearized by partial digestion with EcoRI. After sequential extractions with phenol and chloroform followed by alcohol precipitation to inactivate the EcoRI the DNA is cut to completion using BamHI, the staggered DNA ends are blunted using E.coli DNA polymerase (Klenow enzyme), phosphorylated XhoI linkers (CCTCGAGG) are ligated to the DNA, which is then cut with XhoI restriction enzyme and the 2 kb fragment containing the hygromycin-B resistance gene is purified by gel electrophoresis. The staggered XhoI ends are compatible with SalI ends and DNA is ligated into the expression vectors as described above for pNE-neo and pNE-gpt, yielding pSVG-tPA-hph and pMG-BF-hph (7.6 kb; Fig. 18), respectively.

## C) Transformation of pSVG-tPA-neo and pSVG-tPA-hph

The transfection procedure outlined in Example 3A with the following modifications is used : Alpha-MEM plus nucleosides with undialyzed medium is used to propagate DUKXB1 cells and 1 mg/ml Geneticin (GIBCO, for pSVG-tPA-neo and pMG-BF-neo) or 0.2 mg/ml hygromycin-B (Calbiochem, for pSVG-tPA-hph and pMG-BF-hph) are used to select transformants. Of the resulting colonies analyzed at least 95 % express t-PA at levels equivalent to those produced by pCGA31-dhfr-transformed CHO cells (cf. Example 3G).

## D) Transfection of pSVG-tPA-gpt

Thymidine-kinase-minus L-M cells (ATCC CCL 1.3) are obtained from the American Type Culture Collection and used as host. Transfection is done according to the procedure described in Example 3A with the following modifications: Cells are grown in DMEM with 10 % fetal calf serum. GPT[+] cells are selected by addition of hypoxanthine (15 μg/ml), xanthine (250 μg/ml) and mycophenolic acid (Calbiochem, 2 μg/ml) to the growth medium. Alternatively, GPT[+] CHO-K1 (ATCC CCL 61) cells are selected in MEM-alpha medium with hypoxanthine (15 μg/ml), xanthine (250 μg/ml) and mycophenolic acid (Calbiochem, 2 μg/ml). Of the resulting colonies analyzed at least 95 % express human t-PA as assayed by the fibrin plate assay (see Example 3B).

## E) Transfection of pMG-tPA-tk

Thymidine-kinase-minus L-M cells (ATCC CCL 1.3) are obtained from the American Type Culture Collection and used as host. Cells are grown in DMEM with 10 % fetal calf serum. TK[+] cells are selected in HAT medium: DMEM with hypoxanthin ($10^{-4}$ M), aminopterin ($10^{-7}$ M) and thymidin ($4 \times 10^{-5}$ M). Of the resulting colonies analyzed at least 95 % express human t-PA as assayed by the fibrin plate assay.

## F) Transfection of IgE-BF expression plasmids

Transfection procedures are the same as for the corresponding t-PA expression plasmids, meaning for pMG-BF-neo as for pSVG-tPA-neo, for pMG-BF-hph as for pSVG-tPA-hph, for pMG-BF-gpt as for pSVG-tPA-gpt and for pMG-BF-tk as for pSVG-tPA-tk. Of each transfection 48 colonies are picked, amplified in 24-well microtiter plates and at confluency tested for production of IgE-BF as described in Example 4B. In each of the four different transfection experiments more than 95 % of all cultures produce at least 5 ng/ml of IgE-BF demonstrating that the vector system is operable with different selection markers in different host cell types.

## Example 6: Plasmids expressing sc-uPA (single-chain urokinase) cDNA

Basis for these plasmids is pcUK176 (6.3 kb; Fig. 19), a construct containing a cDNA [DNA sequence in W.E. Holmes et al., Biotechnology 3, 923-929 (1985)] encoding human urokinase cDNA cloned into pUN121 [B. Nilsson et al., Nucl.Acids Res. 11, 8019-8030 (1983)]. pcUK176 is digested with SmaI and AhaIII, phosphorylated SacI linkers are ligated to the resulting blunt termini of the DNA, excess linkers are removed by cutting with SacI restriction enzyme and the 2.25 kb fragment containing the u-PA coding sequence is purified using agarose gel electrophoresis. 10 µg pCGA44 DNA (Example 1D, Fig. 4) is cut to completion with SacI, the linear DNA is purified by gel electrophoresis, followed by extractions with phenol and chloroform. Finally the DNA is precipitated with ethanol, redissolved in TE and 10 ng of the DNA is ligated to 30 ng of the u-PA DNA fragment described above. Upon transformation of E.coli plasmid pBR3a (10.5 kb; Fig. 19) is obtained.

A dhfr-expressing derivative of pBR3a is constructed by cutting 10 µg pBR3a DNA to completion with SalI, purifying the linear DNA, ligating 10 ng thereof to 10 ng of the dhfr-containing XhoI-SalI fragment of pDHFR2911 (cf. Example 1E), which after transformation into E.coli yields tetracyclin-resistant plasmid pBR3a-dhfr (12.7 kb).

In order to transform dhfr⁻ CHO cells, the procedure outlined in Example 3A is followed with the following modifications. A selective mediium (MEM-alpha without nucleosides with dialyzed serum and optionally 1 mg/ml Geneticin, GIBCO) is used. Of the resulting dhfr⁺ colonies analyzed at least 95 % express human u-PA as assayed by the casein and fibrin plate assays (cf. Example 3B). The identity of the product as urokinase is confirmed in that 3 µl rabbit anti-urokinase antibody (raised against Serono urokinase) inhibits halo formation, whereas anti-tPA serum and control rabbit serum do not. When assayed fluorometrically (cf. Example 3C) plasminogen activator activity is at least 10 times higher than in conditioned media from untransformed DUKXB1 cells or cells transformed by a plasmid that does not code for a plasminogen activator.

- 47 -

Deposition of microorganisms

The following microorganisms were deposited on October 23, 1987 at
the Deutsche Sammlung von Mikroorganismen (DSM), Grisebachstrasse 8,
D-3000 Göttingen (FRG).

| microorganism | accession number |
| --- | --- |
| E.coli HB101/pSV2911neo | DSM 4292 |
| E.coli HB101/pDHFR2911 | DSM 4293 |
| E.coli HB101/pCGA26 | DSM 4296 |
| E.coli HB101/pcUK176 | DSM 4290 |

CLAIMS for the contracting states BE, DE, FR, GB, IT,
NL, SE, CH, LU

1. A recombinant DNA molecule comprising a series of functional elements in the following order: (a) an enhancer/promoter unit linked operatively to (b) a DNA segment which can be transcribed into a mRNA having coding capacity for a desired protein product and comprising signals for polyadenylation and for efficient transportation to the cytoplasm, followed by a DNA segment (c) consisting of a promoter unit $(c_1)$ operatively linked to a DNA subsegment $(c_2)$ which can be transcribed into the mRNA of a selection marker gene, wherein said promoter unit $(c_1)$ lacks a functional enhancer but is capable of being stimulated by the enhancer located in (a) due to the absence of an enhancer stop DNA sequence between (a) and $(c_1)$.

2. A DNA molecule according to claim 1 in which unit $(c_2)$ encodes an easily identifiable marker protein taken from the group of antibiotic resistance proteins, enzymes suppressing cellular auxotrophy or producing coloured substances from substrates with no color or a different color.

3. A DNA molecule according to claim 1 in which the DNA subsegment $(c_2)$ is of prokaryotic origin.

4. A DNA molecule according to claim 1 in which the DNA subsegment $(c_2)$ is of eukaryotic origin.

5. A DNA molecule according to claim 3 in which the promoter/ enhancer element a. is preceded by an enhancer-stop sequence.

6. A DNA molecule according to claim 5 in which the enhancer-stop sequence consists of DNA of prokaryotic origin.

7. A DNA molecule according to claim 4 in which the promoter/ enhancer element a. is preceded by an enhancer-stop DNA sequence and in which the DNA segment (c) is followed by an enhancer-stop DNA sequence.

8. A DNA molecule according to claim 7 in which the enhancer-stop sequences consist of DNA of prokaryotic origin.

9. A DNA molecule according to claim 1 in which the marker gene ($c_2$) encodes the enzyme dihydrofolate reductase.

10. A recombinant DNA molecule according to claim 1 in which the DNA sequence of unit b. encodes a desired protein taken from the group of serum proteins, polypeptide hormones, hormone receptors, lymphokines, interferons, enzymes, enzyme inhibitors, analgesic polypeptides, glycoproteins, immunoglobulins, immunoglobulin-binding proteins, antigenic proteins, vaccine proteins, plasminogen activators or a precursor to the said desired protein.

11. A recombinant DNA molecule according to claim 1 in which the promoter/enhancer (a) comprises a promoter and enhancer taken from a gene selected from the group consisting of actin genes, metallothionein genes, other cellular genes, SV40 early gene, herpes virus genes, human cytomegalovirus major immediate early gene, mouse cytomegalovirus major immediate early gene and other viral genes.

12. A recombinant DNA molecule according to claim 1 in which the promoter ($c_1$) is a promoter or comprises a promoter fragment taken from a gene selected from the group consisting of the SV40 early gene, major late adenovirus gene, herpes thymidine kinase gene, human cytomegalovirus major immediate early gene, mouse cytomegalovirus major immediate early gene, cellular genes and metallothionein genes.

13. A host transformed by a DNA molecule according to claim 1.

14. A host according to claim 13 in which said host is taken from the group consisting of E. coli, other bacteria, yeasts, fungi, green plants and animal cells.

15. A host according to claim 13 in which said host is a Chinese hamster ovary cell line.

16. A host according to claim 13 in which said host lacks the enzyme dihydrofolate reductase.

17. A method of producing a desired protein comprising culturing the transformed host according to claim 13 and isolating said protein.

18. A method according to claim 17 comprising culturing mixed clones of transformed host cells.

CLAIMS for the contracting states ES and GR

1. A method of producing a recombinant DNA molecule comprising a series of functional elements in the following order: (a) an enhancer/ promoter unit linked operatively to (b) a DNA segment which can be transcribed into a mRNA having coding capacity for a desired protein product and comprising signals for polyadenylation and for efficient transportation to the cytoplasm, followed by a DNA segment (c) consisting of a promoter unit ($c_1$) operatively linked to a DNA subsegment ($c_2$) which can be transcribed into the mRNA of a selection marker gene, wherein said promoter unit ($c_1$) lacks a functional enhancer but is capable of being stimulated by the enhancer located in (a) due to the absence of an enhancer stop DNA sequence between (a) and ($c_1$), comprising linking said functional elements to a vector DNA such that they are arranged in the predetermied order.

2. A method according to claim 1 comprising a recombinant DNA molecule in which unit ($c_2$) encodes an easily identifiable marker protein taken from the group of antibiotic resistance proteins, enzymes suppressing cellular auxotrophy or producing coloured substances from substrates with no color or a different color.

3. A method according to claim 1 comprising a recombinant DNA molecule in which the DNA subsegment of unit ($c_2$) is of prokaryotic origin.

4. A method according to claim 1 comprising a recombinant DNA molecule in which the DNA subsegment of unit ($c_2$) is of eukaryotic origin.

5. A method according to claim 3 in which the promoter/enhancer element a. is preceded by an enhancer-stop sequence.

6. A method according to claim 5 in which the enhancer-stop sequence consists of DNA of prokaryotic origin.

7. A method according to claim 4 in which the promoter/enhancer element a. is preceded by an enhancer-stop DNA sequence and in which the DNA segment (c) is followed by an enhancer-stop DNA sequence.

8. A method according to claim 7 in which the enhancer-stop sequences consist of DNA of prokaryotic origin.

9. A method according to claim 1 in which the marker gene $(c_2)$ encodes the enzyme dihydrofolate reductase.

10. A method according to claim 1 in which the DNA sequence of unit b. encodes a desired protein taken from the group of serum proteins, polypeptide hormones, hormone receptors, lymphokines, interferons, enzymes, enzyme inhibitors, analgesic polypeptides, glycoproteins, immunoglobulins, immunoglobulin-binding proteins, antigenic proteins, vaccine proteins, plasminogen activators or a precursor to the said desired protein.

11. A method according to claim 1 in which the promoter/enhancer (a) comprises a promoter and enhancer taken from a gene selected from the group consisting of actin genes, metallothionein genes, other cellular genes, SV40 early gene, herpes virus genes, human cytomegalovirus major immediate early gene, mouse cytomegalovirus major immediate early gene and other viral genes.

12. A method according to claim 1 in which the promoter $(c_1)$ is a promoter or comprises a promoter fragment taken from a gene selected from the group consisting of the SV40 early gene, major late adenovirus gene, herpes thymidine kinase gene, human cytomegalovirus major immediate early gene, mouse cytomegalovirus major immediate early gene, cellular genes and metallothionein genes.

13. A method of producing a transformed host comprising a recombinant DNA molecule comprising a series of functional elements in the following order: (a) an enhancer/ promoter unit linked operatively to (b) a DNA segment which can be transcribed into a mRNA having coding capacity for a desired protein product and comprising signals for polyadenylation and for efficient transportation to the cytoplasm, followed by a DNA segment (c) consisting of a promoter unit $(c_1)$ operatively linked to a DNA subsegment $(c_2)$ which can be transcribed into the mRNA of a selection marker gene, wherein said promoter unit $(c_1)$ lacks a functional enhancer but is capable of being stimulated by the enhancer located in (a) due to the absence of an enhancer stop DNA sequence between (a) and $(c_1)$, comprising transforming a host with said recombinant DNA molecule.

14. A method of producing a desired protein comprising culturing a host transformed by a recombinant DNA molecule comprising a series of functional elements in the following order: (a) an enhancer/ promoter unit linked operatively to (b) a DNA segment which can be transcribed into a mRNA having coding capacity for a desired protein product and comprising signals for polyadenylation and for efficient transportation to the cytoplasm, followed by a DNA segment (c) consisting of a promoter unit $(c_1)$ operatively linked to a DNA subsegment $(c_2)$ which can be transcribed into the mRNA of a selection marker gene, wherein said promoter unit $(c_1)$ lacks a functional enhancer but is capable of being stimulated by the enhancer located in (a) due to the absence of an enhancer stop DNA sequence between (a) and $(c_1)$, and isolating said protein.

15. A method according to claim 14 comprising culturing mixed clones of transformed host cells.

FO 7.4/UL/cc*/gb*

Figure 1

Figure 2

Figure 3

Figure 4

Figure 5

0 271 003

Figure 6

Figure 7

Figure 8

**pCL2**

Hind3
Pst1
SP6 promoter / G/C-tail
Rsa1
Rsa1
Nco1
SP6-transcript
IgE-receptor
T7 transcript
1.25 kb
Hind3
Bgl2
Rsa1
Rsa1
AMP
Pvu1
Nco1
Hinc2
G/C-tail
Pst1
Hinc2
Xba1
BamH1
EcoR1
T7 promoter
pBRori

**pGEM-TM-1**

Hind3
Sal1 < Pst1
Sma1 < BamH1 < Xba1
EcoR1 < Sac1
pBRori
Sph1
T7-promoter
SP6-promoter
AMP
Pvu1

HincII + RsaII
purify 1.25 kb fragment

HincII, CIP

partial cut with HindIII
complete digest with SalI
purify 1.25 kb fragment
(cf. figs. 10 and 14)

**pCAL3**

Hind3
Pst1
Sal1
SP6 promoter
Nco1
Bgl2
Hind3
Dde1
IgE-receptor
1.25 kb
0.7 kb
Rsa1
Nco1
Rsa1
Xba1
AMP
BamH1
Pvu1
EcoR1
T7 promoter
pBRori

HindIII + XbaI
purify 0.75 kb fragment
DdeI, purify 0.7 kb fragment
(cf. fig. 11)

Figure 9

0 271 003

Figure 10

Figure 11

pSVG-BF

Ncol < EcoRI
72 bp
72 bp
SVorI < Ncol
Bgl2 < Ncol < Hind3
IgE-BF
Ncol
mRNA
SPLD < BamHI < Xbal
Ncol
SPLA
EcoRI
Bgl2
polyA
Sall
AMP
PvuI
pBRorI

pSVG-BF-dhfr

XbaI
BamHI
SPLD
SPLA
EcoRI
Bgl2
polyA
IgE-BF
mRNA
AdMLP
SPLD
Hind3
Bgl2
SPLA
72 bp
72 bp
SVorI
Hind3
Bgl2
EcoRI
AMP
PvuI
DHFR
mRNA
Tet
Hind3/
EcoRI
BamHI
polyA
Sall
BamHI
pBRorI

Sall, CIP: ligate to
2.2 kb XhoI-Sall fragment
of pDHFR2911 (cf. fig. 5)

XbaI + HindIII,
purify 4.3 kb fragment

pCAL3 cut with HindIII + XbaI,
purify 0.75 kb fragment,
cut with DdeI, purify 0.7 kb fragment
(cf. fig. 9)

Synthetic, phosphorylated
DNA, HindIII-DdeI

pSVG-ER
4.3 kb

Bgl2
Hind3
DdeI
IgE-receptor
72 bp
72 bp
SVorI
Hind3
Sall
EcoRI
mRNA
XbaI
BamHI
SPLD
SPLA
EcoRI
Bgl2
polyA
AMP
PvuI
Sall
pBRorI

Figure 12

# Figure 13

Figure 14

0 271 003

Figure 15

Figure 16

# Figure 17

Figure 18

Figure 19